# EUROPEAN PATENT APPLICATION

(11) **EP 4 082 425 A1**
(43) Date of publication of application: **02.11.2022**
(21) Application number: 20905101.0
(22) Date of filing: 04.12.2020
(51) Int. Cl.: A61B 5/00, A61B 5/349, A61M 21/02, A61M 21/00

(54) **BIOFEEDBACK DEVICE USING ELECTROCARDIOGRAM, AND CONTROL METHOD THEREFOR**

(30) Priority: 24.12.2019 KR 20190174205
(71) Applicant: Mezoo Co., Ltd., Wonju-si, Gangwon-do 26354 (KR)
(72) Inventor: PARK, Jung Hwan, Wonju-si, Gangwon-do 26494 (KR); CHO, Sung Pil, Wonju-si, Gangwon-do 26494 (KR); SONG, Mi Hye, Wonju-si, Gangwon-do 26494 (KR); SHIN, Jae Yeon, Wonju-si, Gangwon-do 26424 (KR); SHIM, Hun, Wonju-si, Gangwon-do 26392 (KR); PARK, Jun Hyun, Wonju-si, Gangwon-do 26352 (KR)
(74) Representative: Schmitt-Nilson Schraud Waibel Wohlfrom Patentanwälte Partnerschaft mbB
(86) International application number: PCT/KR2020/017689
(87) International publication number: WO 2021/132933

(57) **Abstract**

The present invention relates to a biofeedback device using an electrocardiogram, and the biofeedback device using an electrocardiogram may include: a signal acquisition unit acquiring an electrocardiogram signal of a user from an electrocardiograph; an analysis unit analyzing the acquired electrocardiogram signal and providing a state diagnosis result of the user as an analysis result; and a control unit controlling at least one stimulation unit among a plurality of stimulation units for state control of the user based on the state diagnosis result, and a stimulation corresponding to the at least one stimulation unit may be provided to the user by the control of the control unit.

## Description

### [Technical Field]

The present invention relates to a biofeedback device using an electrocardiogram (ECG) and a method for controlling the same.

### [Background Art]

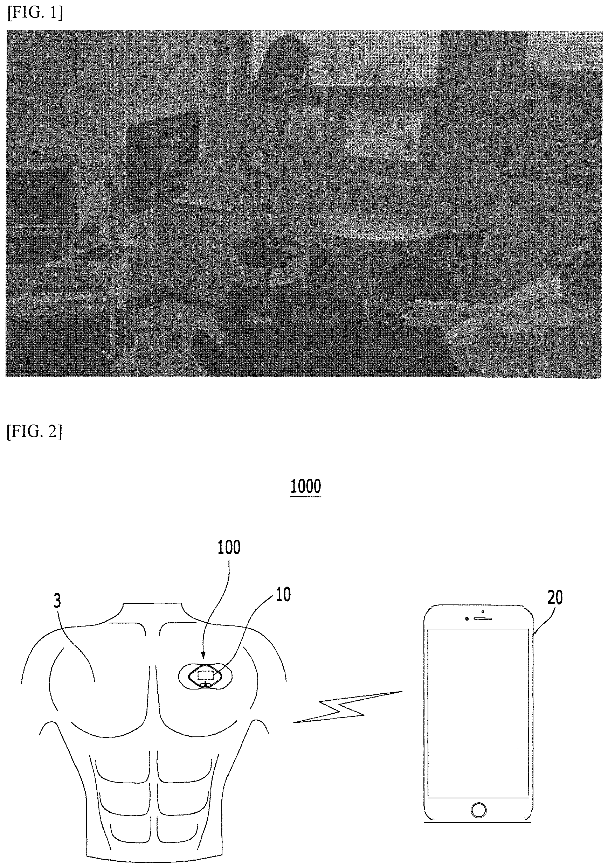

FIG. 1 is a diagram for describing a biofeedback in the related art.

Referring to FIG. 1, biofeedback means technology that measures the mind and body response using a special device and feeds back and announces the measured information to a patient in a recognizable form such as sound, light, or graph to be used for training the patient to self-regulate his/her relaxation or tension.

Biofeedback treatment means that an ability of accurately realizing the change in one's own physiological activity and self-recognizing and controlling while self-checking various involuntary physiological activities such as being controlled by the autonomic nervous system, such as blood pressure, heart rate, skin temperature, muscle tone, respiration, and skin conduction is trained. The biofeedback technology is being used as a treatment for reducing pain, relieving stress, mental stability, and promoting overall health.

In general, in the biofeedback of the related art, in a state in which the user conveniently sits or lies in a specific space in which a biofeedback device is provided, electrodes (or sensors) for measuring a physiological response (a biological signal) of the user are attached to a body of the user and the physiological response is checked based on a response of the signal applied to the corresponding electrode, and as a result, a procedure is performed.

However, the biofeedback technology of the related art is limited in space in using the technology as illustrated in FIG. 1, the configuration is complicated due to a plurality of wires connected to the electrode, and there is inconvenience such as limitation in movement/motion of the user.

The background art of the present invention is disclosed in Korean Patent Registration No. 10-1990383.

### [Disclosure]

### [Technical Problem]

The present invention is to solve the problem, and an object of the present invention is to provide a biofeedback device using an electrocardiogram and a method for controlling the same which may solve problems such as limitation of the biofeedback technology of the related art in a space, a complicated configuration due to a plurality of wires connected to an electrode, and limitation of movement/motion of a user.

However, a technical object to be achieved by an exemplary embodiment of the present invention is not limited to the technical objects and there may be other technical objects.

### [Technical Solution]

As a technical means for achieving the technical problem, according to an embodiment of the present invention, a biofeedback device using an electrocardiogram may include: a signal acquisition unit acquiring an electrocardiogram signal of a user from an electrocardiograph; an analysis unit analyzing the acquired electrocardiogram signal and providing a state diagnosis result of the user as an analysis result; and a control unit controlling at least one stimulation unit among a plurality of stimulation units for state control of the user based on the state diagnosis result, and a stimulation corresponding to the at least one stimulation unit may be provided to the user by the control of the control unit.

Further, the plurality of stimulation units may be provided in a type embedded in the biofeedback device and may include at least two of a vibration stimulation unit capable of providing a vibration stimulation, a sound stimulation unit capable of providing a sound stimulation, a light stimulation unit capable of providing a light stimulation, and an odor stimulation unit capable of providing an odor stimulation.

Further, when there is a waveform which satisfies a predetermined feature condition within a waveform of the acquired electrocardiogram signal, the analysis unit may identify that a signal corresponding to the waveform satisfying the predetermined feature condition is an abnormal suspicious electrocardiogram signal.

Further, the analysis unit may finally identify whether the abnormal suspicious electrocardiogram signal is an abnormal electrocardiogram signal based on a comparison of a similarity between waveform data of a plurality of abnormal electrocardiogram signals prestored in a database and waveform data of the abnormal suspicious ECG signal to provide the state diagnosis result.

Further, the analysis unit may identify a heart rate of the user by analyzing the electrocardiogram signal, and the control unit may differently control a stimulation providing type of the stimulation provided from the at least one stimulation unit by considering the identified heart rate and control the stimulation providing type of the stimulation provided by the at least one stimulation unit to be changed from a first stimulation providing type to a second stimulation providing type.

Further, the odor stimulation unit may include a plurality of fragrance storage units storing a plurality of types of fragrance materials, and an injection nozzle unit connected to the plurality of fragrance storage units and injecting any one of fragrance materials stored in the plurality of fragrance storage units by the control of the control unit.

Further, the control unit may control an operation of the injection nozzle unit to inject a fragrance material stored in any one fragrance storage unit among the plurality of fragrance storage units when the heart rate satisfies the predetermined reference, and control the operation of the injection nozzle unit to inject the fragrance material stored in any one fragrance storage unit other than the any one fragrance storage unit among the plurality of fragrance storage units when the heart rate does not satisfy the predetermined reference.

Meanwhile, according to an embodiment of the present invention, a control method of a biofeedback device using an electrocardiogram may include: acquiring, by a signal acquisition unit, an electrocardiogram signal of a user from an electrocardiograph; analyzing, by an analysis unit, the acquired electrocardiogram signal and providing a state diagnosis result of the user as an analysis result; and controlling, by a control unit, at least one stimulation unit among a plurality of stimulation units for the biofeedback for state control of the user based on the state diagnosis result, and a stimulation corresponding to the at least one stimulation unit may be provided to the user by the controlling.

The problem-solving means is just exemplary, and should not be interpreted as an intention of limiting the present invention. In addition to the exemplary embodiment, an additional embodiment may exist in drawings and a detailed description of the present invention.

The above-mentioned aspects are merely exemplary and should not be construed as limiting the present invention. In addition to the above-described exemplary embodiments, additional exemplary embodiments may exist in the drawings and detailed description of the invention.

### [Advantageous Effects]

According to the technical solution of the present invention, it is possible to solve problems of the conventional biofeedback technology (i.e., the problems such as the limitation in the space, the complicated configuration due to the plurality of wires connected to the electrode, and the imitation of the movement/motion of the user) by providing a biofeedback device using an electrocardiogram.

According to the technical solution of the present invention, it is possible to provide a biofeedback device using an electrocardiogram, which is easy to carry, convenient of use, and enables an effective treatment effect to be provided.

However, an effect which can be obtained in the present invention is not limited to the effects, and there may be other effects.

### [Description of Drawings]

FIG. 1 is a diagram for describing a biofeedback in the related art.
FIG. 2 is a diagram schematically illustrating a configuration of a biofeedback system using an electrocardiogram according to an embodiment of the present invention.
FIG. 3 is a block diagram illustrating a schematic configuration of a biofeedback device using an electrocardiogram according to an embodiment of the present invention.
FIG. 4 is a diagram illustrating a waveform example of an electrocardiogram signal obtained by an electrocardiogram in the biofeedback device using the electrocardiogram according to an embodiment of the present invention.
FIG. 5 is a diagram for describing a process in which an analysis unit of the biofeedback device using the electrocardiogram calculates a similarity between waveform data of an abnormal suspicious ECG signal and waveform data of an abnormal ECG signal according to an embodiment of the present invention.
FIG. 6 is a schematic block diagram of the electrocardiograph included in the biofeedback device using the electrocardiogram according to an embodiment of the present invention.
FIG. 7 is a diagram schematically illustrating an attachment direction of the electrocardiograph included in the biofeedback device using the electrocardiogram according to an embodiment of the present invention.
FIG. 8 is a block diagram illustrating a schematic configuration of a user terminal included in the biofeedback system using the electrocardiogram according to an embodiment of the present invention.
FIG. 9 is a diagram illustrating an example in which a guide information providing unit of the user terminal included in the biofeedback system using the electrocardiogram according to an embodiment of the present invention outputs guide information.
FIG. 10 is a diagram for describing an electrocardiogram lead guide technology using the electrocardiograph and the user terminal included in the biofeedback system using the electrocardiogram according to an embodiment of the present invention.
FIG. 11 is an operation flowchart for a control method of a biofeedback device using an electrocardiogram according to an embodiment of the present invention.
FIG. 12A is a diagram for describing a biofeedback principle by the biofeedback device using the electrocardiogram according to an embodiment of the present invention.
FIG. 12B is a diagram for describing a case of feeding back a vibration stimulation as a biofeedback in the biofeedback device using the electrocardiogram according to an embodiment of the present invention.
FIG. 12C is a diagram for describing a case of feeding back a sound stimulation as the biofeedback in the biofeedback device using the electrocardiogram according to an embodiment of the present invention.
FIG. 12D is a diagram for describing a case of feeding back a light stimulation as the biofeedback in the biofeedback device using the electrocardiogram according to an embodiment of the present invention.
FIG. 12E is a diagram for describing a case of feeding back an odor stimulation as the biofeedback in the biofeedback device using the electrocardiogram according to an embodiment of the present invention.

### [Best Mode]

Hereinafter, exemplary embodiments of the present disclosure will be described in detail so as to be easily implemented by those skilled in the art, with reference to the accompanying drawings. However, the present invention may be implemented in various different forms and is not limited to exemplary embodiments described herein. In addition, in the drawings, in order to clearly describe the present invention, a part not related to the description is not omitted and like reference numerals designate like elements throughout the specification.

Throughout the specification, when it is described that a part is "connected" with another part, it means that the certain part may be "directly connected" with another part and the parts "electrically connected" or "directly connected" to each other with a third element interposed therebetween as well.

Throughout the specification, it will be understood that when a member is referred to as being "on", "in upper portion", "in upper end", "below", "in lower portion", and "in lower end" another member, it may be directly on the other member or intervening members may also be present.

Throughout the specification, unless explicitly described to the contrary, a case where any part "includes" any component will be understood to imply the inclusion of stated components but not the exclusion of any other component.

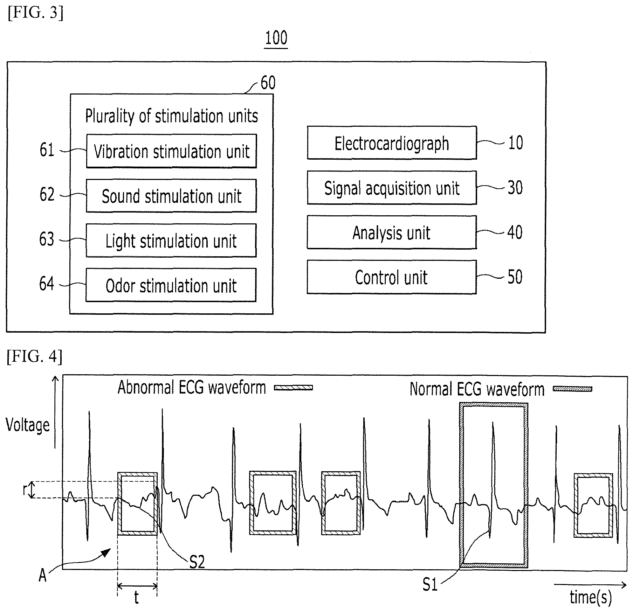

FIG. 2 is a diagram schematically illustrating a configuration of a biofeedback system 1000 using an electrocardiogram according to an embodiment of the present invention and FIG. 3 is a block diagram illustrating a schematic configuration of a biofeedback device 100 using an electrocardiogram according to an embodiment of the present invention.

Hereinafter, for convenience of description, the biofeedback system 1000 using an electrocardiogram according to an embodiment of the present invention is referred to as the present system 1000, and the biofeedback device 100 using an electrocardiogram according to an embodiment of the present invention will be referred to as the present device 100.

Referring to FIGS. 2 and 3, the present system 1000 may include the present device 100 and a user terminal 20.

The present device 100 may obtain an electrocardiogram signal of a user 3 from an electrocardiograph 10 included in the present device 100, and control at least one simulation unit among a plurality of simulation units 60 included in the present device 100 based on an analysis result of analyzing the obtained electrocardiogram signal (this may be expressed differently as a state diagnosis result).

The present device 100 may provide the state analysis result to the user terminal 20 as an example through the control unit 50. Further, an operation of the present device 100 may be controlled by the user terminal 20 (i.e., remotely controlled) as an example.

Data (information) may be transmitted and received between the present device 100 and the user terminal 20 through a network.

An example of the network for sharing information between the present device 100 and the user terminal 20 may include a 3rd Generation Partnership Project (3GPP) network, a Long Term Evolution (LTE) network, a 5G network, a World Interoperability for Microwave Access (WIMAX) network, wired/wireless Internet, a Local Area Network (LAN), Wireless Local Area Network (LAN), a Wide Area Network (WAN), a Personal Area Network (PAN), a Bluetooth network, a WiFi network, a near field communication (NFC) network, a satellite broadcasting network, an analog broadcasting network, a Digital Multimedia Broadcasting (DMB) network, etc., but is not limited thereto.

The user terminal 20 may mean a device which interlocks with the present device 100 through the network. The user terminal 20 may include all types of wired/wireless communication devices including Personal Communication System (PCS), Global System for Mobile Communications (GSM), Personal Digital Cellular (PDC), Personal Handyphone System (PHS), Personal Digital Assistant (PDA), International Mobile Telecommunication (IMT)-2000, Code Division Multiple Access (CDMA)-2000, W-Code Division Multiple Access (W-CDMA), Wireless Broadband Internet (Wibro) terminals, smartphones, smart pads, tablet PCs, a notebook computer, a wearable device, a desktop PC, etc., and is not limited thereto.

The present device 100 may be provided as a patch type that may be attached to a part of the body of the user 3, a wearable type (for example, a watch type wearable on a wrist of the user, etc.) wearable on a part of the body of the user 3, and a type which may be embedded in various articles, etc., but is not limited thereto. Here, various articles may be, for example, yoga articles, massage chairs, car steering wheels, and the like.

The present device 100 may include an electrocardiograph 10, a signal acquisition unit 30, an analysis unit 40, a control unit 50, and a plurality of stimulation units 60.

As an example, the electrocardiograph 10 may be provided as the type embedded in the present device 100. That is, the present device 100 may include the electrocardiograph 10.

In an example of the present invention, it is described that the electrocardiograph 10 is a component (i.e., a component provided in the type embedded in the present device) included in the present device 100 but is not limited thereto.

As another example, the electrocardiograph 10 may be provided as a component separately separated from the present device 100. As such, when the electrocardiograph 10 and the present device 100 are provided as the components separated from each other, the present device 100 may acquire an electrocardiogram signal measured (sensed) by the electrocardiograph 10 from the electrocardiograph 10 through the network by wireless communication.

The electrocardiograph 10 may measure the electrocardiogram (ECG) signal of the user 3 and provide the measured ECG signal to the signal acquisition unit 30.

The signal acquisition unit 30 may acquire the ECG signal of the user 3 from the electrocardiograph 10 wiredly/wirelessly. In the present invention, the user 3 may be, for example, a patient, but is not limited thereto.

The analysis unit 40 may analyze the ECG signal acquired by the signal acquisition unit 30 and may provide a state diagnosis result of the user 3 as an analysis result. Here, the state diagnosis result may include whether a heart state is normal/abnormal but is not limited thereto. The state diagnosis result may also include data of the ECG signal acquired by the signal acquisition unit 30.

Here, the heart state being normal may be expressed differently as a heath state of the user being a stable state, and the heart state being abnormal may be expressed differently as the health state of the user being an unstable state.

That is, the analysis unit 40 analyzes the ECG signal acquired by the signal acquisition unit 30 to identify (analyze) whether the heart state of the user 3 is normal (i.e., whether the health state is the stable state) or abnormal (i.e., whether the health state is the unstable state) as an example as the state diagnosis result.

When there is a waveform which satisfies a predetermined feature condition within a waveform of the ECG signal acquired by the signal acquisition unit 30, the analysis unit 40 may primarily identify that a signal corresponding to the waveform satisfying the predetermined feature condition is an abnormal suspicious ECG signal. Here, the predetermined feature condition may mean a condition for a feature that a signal belonging to a threshold range is generated for a predetermined time.

In other words, the analysis unit 40 may primarily identify whether the abnormal suspicious ECG signal suspected as an abnormal ECG signal is generated according to whether there is a waveform in which the signal belonging to the threshold range is generated for the predetermined time among the waveforms of the ECG signal acquired by the electrocardiograph 10. This may be more easily appreciated with reference to FIG. 4. Here, as an example, the threshold range may mean a range set for an amplitude of the waveform of the ECG signal acquired by the electrocardiograph 10.

FIG. 4 is a diagram illustrating a waveform example of an electrocardiogram signal acquired (measured) by an electrocardiograph 10 in the biofeedback device 100 using the electrocardiogram according to an embodiment of the present invention.

As the waveform of the ECG signal acquired (measured) by the electrocardiograph 10 illustrated in FIG. 4, a waveform S1 of the normal ECG signal as a waveform S1 which does not satisfy the predetermined feature condition and a waveform S2 of the abnormal suspicious ECG signal suspected as the abnormal ECG signal as a waveform S2 which satisfies the predetermined feature condition are illustrated.

Referring to FIG. 4, the analysis unit 40 may identify whether the waveform S2 satisfying a feature (waveform feature) that a signal belonging to a threshold range r is generated for a predetermined time t is present (generated) as the predetermined feature condition among the waveforms of the ECG signal acquired by the electrocardiograph 10. In this case, when the waveform S2 satisfying the predetermined feature condition is present, the analysis unit 40 may primarily identify a signal (electric signal) corresponding to the waveform S2 satisfying the predetermined feature condition as the abnormal suspicious ECG signal which may be the abnormal ECG signal represented as the heart state of the user 3 is not good.

In other words, when there is the waveform S2 in which the signal belonging to the threshold range r is generated for the predetermined time t among the waveforms of the acquired ECG signal, the analysis unit 40 may primarily identify a signal corresponding to the waveform, i.e., the signal corresponding to the waveform S2 satisfying the predetermined feature condition as the abnormal suspicious ECG signal which may be the abnormal ECG signal. That is, in the drawing of FIG. 4, a signal corresponding to the waveform S2 corresponding to interval A may be primarily identified as the abnormal suspicious ECG signal.

Here, the threshold range r may mean a predetermined range for a value (or a value of the electric signal of the ECG signal) of the ECG signal measured (acquired) through the electrocardiograph 10. In this case, in FIG. 4, a case where the electric signal of the ECG signal acquired from the electrocardiograph 10 is a voltage signal is illustrated, and in this case, the threshold range r may be exemplarily set to a range of a first voltage value or more and a second voltage value or less.

Therefore, when the electric signal having the first voltage value or more and the second voltage value or less among the ECG signals acquired from the electrocardiograph 10 is generated for the predetermined time t, the analysis unit 40 may primarily identify the corresponding signal as the abnormal suspicious ECG signal.

According to this, when the signal of the waveform S2 satisfying the predetermined feature condition is generated from the ECG signal (electric signal) acquired by the electrocardiograph 10, the analysis unit 40 may not immediately identify that the signal corresponding to the waveform S2 is the abnormal ECG signal, but primarily (temporarily) identify the signal corresponding to the waveform S2 as the abnormal suspicious ECG signal which may be the abnormal ECG signal.

After primarily identifying that the signal corresponding to the waveform S2 as the abnormal suspicious ECG signal, the analysis unit 40 may secondarily identify (i.e., finally identify) whether the abnormal suspicious ECG signal is the abnormal ECG signal based on a comparison of a similarity between waveform data of a plurality of abnormal ECG signals prestored in a database (not illustrated) and waveform data of the abnormal suspicious ECG signal. The analysis unit 40 may provide the state diagnosis result based on a secondary identification result.

Specifically, the analysis unit 40 may perform the comparison of the similarity between each of the waveform data of the plurality of abnormal ECG signals prestored in the database (not illustrated) and the waveform data of the abnormal suspicious ECG signal primarily identified above. Through the similarity comparison, the analysis unit 40 may identify whether there is waveform data of the abnormal ECG signal having a similarity to the waveform data of the abnormal suspicious ECG signal which exceeds a threshold similarity among the waveform data of the plurality of abnormal ECG signals.

In this case, when there is at least one waveform data of the abnormal ECG signal having the similarity which exceeds the threshold similarity among the waveform data of the plurality of abnormal ECG signals, the analysis unit 40 may secondarily finally identify that the waveform data of the abnormal suspicious ECG signal is waveform data of an actual abnormal ECG signal. Through such the secondary identification, the analysis unit 40 may finally identify (analyze) that the abnormal ECG signal is generated in the ECG signal acquired by the electrocardiograph 10.

That is, when there is at least one waveform data of the abnormal ECG signal having the similarity which exceeds the threshold similarity in the database (not illustrated) through the similarity comparison in relation to the waveform data of the abnormal suspicious ECG signal which is primary identified, the analysis unit 40 may secondarily (finally) identify that the primarily identified abnormal suspicious ECG signal is the abnormal ECG signal.

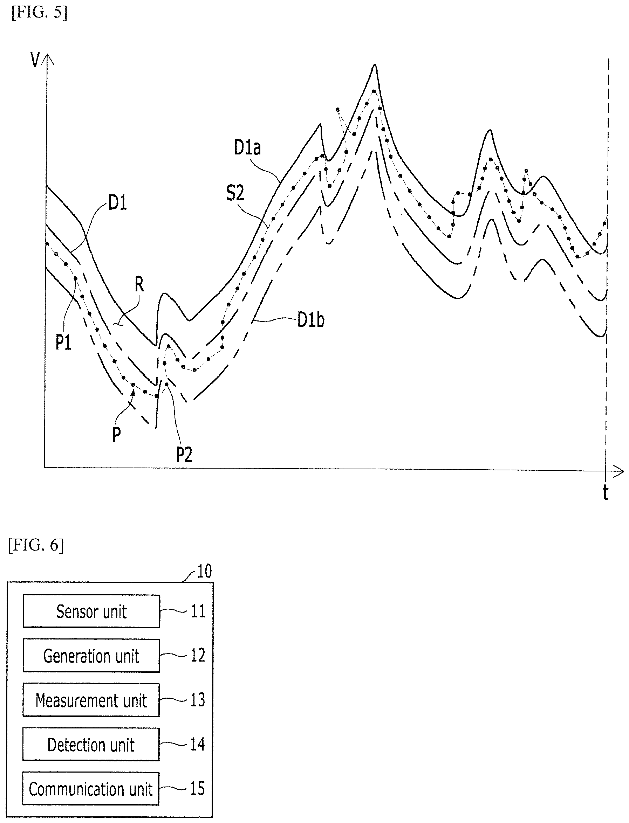

The description of the similarity calculation process may be more easily appreciated with reference to FIG. 5.

FIG. 5 is a diagram for describing a process in which an analysis unit 40 of the biofeedback device 100 using the electrocardiogram calculates a similarity between waveform data of an abnormal suspicious ECG signal and waveform data of an abnormal ECG signal according to an embodiment of the present invention.

In FIG. 5, as an example, S2 represents an example of the waveform data (i.e., the abnormal suspicious ECG signal) of the abnormal suspicious ECG signal primarily identified above. Further, D1 as waveform data (i.e., any one abnormal ECG signal) of any one abnormal ECG signal of the waveform data of the plurality of abnormal ECG signals prestored in the database (not illustrated) represents an example of waveform data (i.e., a first abnormal ECG signal) of the first abnormal ECG signal, as an example.

Hereinafter, for convenience of description, S2 will be referred to as the abnormal suspicious ECG signal and D1 will be referred to as the first abnormal ECG signal. Further, a value (a value of an electric signal corresponding to the first abnormal ECG signal) of the first abnormal ECG signal D1 may be expressed differently as a reference value. In this case, contents described for the first abnormal ECG signal D1 may also be similarly applied to the description of each of the plurality of abnormal EDG signals prestored in the database (not illustrated) in spite of contents omitted below.

Further, D1a represents an upperlimit value which is a value acquired by adding a predetermined error range value (e.g., e%) to the reference value and D1b represents a lowerlimit value which is a value acquired by subtracting the predetermined error range value (e%) from the reference value. Here, a range R of the lower limit value D1b or more and the upperlimit value D1a or less may be referred to as a tolerance range (error tolerance range). In other words, the tolerance range R (error tolerance range) may mean a range in which the value (e.g., the voltage value) of the electric signal belongs to the lowerlimit value D1b or more and the upperlimit value D1a or less.

Referring to FIG. 5, the analysis unit 40 may compare (i.e., calculate) the similarity between the first abnormal ECG signal D1 which is any one of the waveform data of the plurality of abnormal ECG signals stored in the database (not illustrated) and the abnormal suspicious ECG signal S2.

For the similarity comparison, the analysis unit 40 may acquire values of electric signals for a predetermined number (e.g., 100) of sampling points P from the abnormal suspicious ECG signal S2. For example, the analysis unit 40 may acquire the value of the electric signal corresponding to each of 100 sampling points with respect to 100 sampling points P on the abnormal suspicious ECG signal S2.

Here, positions of a predetermined number of sampling points on the abnormal suspicious ECG signal S2 may be determined as positions where the sampling points are arranged at an interval in which a distance between the sampling points is equal with respect to a time (i.e., a predetermined time t) length corresponding to the abnormal suspicious ECG signal S2. That is, the predetermined number of sampling points may be determined as positions separated from each other at an equal interval.

The predetermined number of sampling points P may include a first sampling point P1 located within the tolerance range R and a second sampling point P2 located out of the tolerance range R. That is, the first sampling point P1 may mean a point located in the range of the lower limit value D1b or more and the upperlimit value D1a or less. The second sampling point P2 may mean a point located in a range less than the lower limit value D1b or located a range more than the upperlimit value D1a.

The analysis unit 40 may calculate the number of first sampling points P1 located within the tolerance range among the predetermined number of sampling points P and the number of second sampling points P2 located out of the tolerance range. The analysis unit 40 may calculate the similarity between the first abnormal ECG signal D1 and the abnormal suspicious ECG signal S2 by using a ratio between the number of first sampling points P1 and the number of second sampling points P2.

The analysis unit 40 may calculate the similarity (similarity value) between the first abnormal ECG signal D1 and the abnormal suspicious ECG signal S2 by using Equation 1 below. $S \left(%\right) = N 1 / N \times 100 %$

Here, S(%) represents the similarity (similarity value) between the first abnormal ECG signal D1 and the abnormal suspicious ECG signal S2, N1 represents the number of first sampling points P1 located within the tolerance range, and N represents the number of second sampling points P2 located out of the tolerance range.

As an example, when the number of first sampling points P1 among 100 sampling points P is 90 and the number of second sampling points P2 is 10, the similarity (similarity value) between the first abnormal ECG signal D1 and the abnormal suspicious ECG signal S2 may be 90%.

As such, the analysis unit 40 may perform the comparison of the similarity between the plurality of abnormal ECG signals (i.e., the waveform data of the plurality of ECG signals) prestored in the database (not illustrated) and the suspicious ECG signals S2 (the waveform data of the abnormal suspicious ECG signal). In other words, the analysis unit 40 may calculate a similarity of each of the plurality of abnormal ECG signals to the abnormal suspicious ECG signal S2.

As a result of the similarity comparison (calculation), when there is at least one abnormal ECG signal having a similarity to the abnormal suspicious ECG signal S2, which exceeds the threshold similarity among the plurality of abnormal ECG signals, the analysis unit 40 may secondarily identify that the abnormal suspicious ECG signal S2 is the abnormal ECG signal. When identifying that the abnormal suspicious ECG signal S2 is the abnormal ECG signal, the analysis unit 40 may finally identify that the abnormal ECG signal is generated within the ECG signal acquired by the electrocardiograph 10. Here, the threshold similarity as a value which becomes a reference for identifying whether the abnormal suspicious ECG signal is the abnormal ECG signal may mean a threshold value for the similarity calculated through Equation 1 above. As an example, the threshold similarity may be set to 98%, but is not limited thereto.

In other words, the analysis unit 40 may perform a matching comparison between the waveform data of the abnormal suspicious ECG signal S2 and the waveform data of the plurality of abnormal ECG signals prestored in the database (not illustrated) in order to determine whether is waveform data matching the waveform data of the abnormal suspicious ECG signal S2 among the waveform data of the plurality of abnormal ECG signals prestored in the database (not illustrated).

In this case, when there is at least one waveform data of the abnormal ECG signal matching the waveform data of the abnormal suspicious ECG signal S2 among the waveform data of the plurality of abnormal ECG signals prestored in the database (not illustrated), the analysis unit 40 identifies that the primarily identified abnormal suspicious ECG signal S2 is the secondarily identified abnormal ECG signal to finally identify that the ECG signal of the user 3 is abnormal (i.e., identify that the abnormal ECG signal is generated within the ECG signal acquired by the electrocardiograph).

Here, the waveform data of the abnormal ECG signal matching the waveform data of the abnormal suspicious ECG signal S2 may also mean data for the waveform of the abnormal ECG signal 100% matching the waveform of the abnormal suspicious ECG signal S2, but is not limited thereto, and as described above, may mean data for the waveform of the abnormal ECG signal which belongs to a predetermined similarity range (i.e., having the similarity which exceeds the threshold similarity).

When the analysis unit 40 secondarily identifies (finally identifies) that the primarily identified abnormal suspicious ECG signal is the abnormal ECG signal, the analysis unit 40 may recognize that a current heart state of the user 3 is the abnormal state (i.e., the health state of the user is the unstable state) and provide a state diagnosis result indicating that the current heart state of the user 3 is the abnormal state.

The control unit 50 may control at least one simulation unit among the plurality of simulation units 60 for state control of the user 3 based on the state diagnosis result provided by the analysis unit 40. By the control by the control unit 50, a stimulation corresponding to at least one simulation unit controlled by the control unit 50 may be provided to the user 3 (may be achieved).

As an example, when the state diagnosis result indicating that the heart state of the user 3 is the 'normal' state is provided by the analysis unit 40, the control unit 50 may control at least one simulation unit of the plurality of simulation units 60 to provide a first stimulation providing type of simulation. Meanwhile, when the state diagnosis result indicating that the heart state of the user 3 is the 'abnormal' state is provided by the analysis unit 40, the control unit 50 may control at least one simulation unit of the plurality of simulation units 60 to provide a second stimulation providing type of simulation.

In this case, the second stimulation providing type may mean a type in which a strength of the stimulation is strong, or a time of the stimulation is set to be long relatively as compared with the first stimulation providing type. A specific example thereof may be more easily appreciated through a description to be made.

The plurality of stimulation units 60 may be provided in a type embedded in the present device 100 (the biofeedback device using the ECG).

The plurality of stimulation units 60 may include at least two of a vibration stimulation unit 61 capable of providing a vibration stimulation, a sound stimulation unit 62 capable of providing a sound stimulation, a light stimulation unit 63 capable of providing a light stimulation, and an odor stimulation unit 64 capable of providing an odor stimulation.

Further, the analysis unit 40 may identify a heart rate of the user 3 by analyzing the ECG signal acquired by the electrocardiograph 10.

The control unit 50 may differently control the stimulation providing type of the stimulation provided from at least one stimulation unit by considering the heart rate identified by the analysis unit 40 (or in accordance with the heart rate or in synchronization with the heart rate).

Here, the stimulation providing type may include at least one of the strength of the stimulation, the time of the stimulation, and a pattern of the stimulation.

As a specific example, the control unit 50 as the stimulation providing type of the vibration stimulation provided by the vibration stimulation unit 61 may control at least one of the strength of vibration, a vibration time, and a vibration pattern. The control unit 50 as a stimulation providing type of the sound stimulation provided by the sound stimulation unit 62 may control at least one of the strength (magnitude) of the sound, a sound emission time, and a sound emission pattern. The control unit 50 as a stimulation providing type of the light stimulation provided by the light stimulation unit 63 may control at least one of the strength of the light, a light emission time, and a light emission pattern. The control unit 50 as a stimulation providing type of the odor stimulation provided by the odor stimulation unit 64 may control at least one of the strength of the odor (a discharge amount of the odor), a discharge time of the odor, and a discharge pattern of the odor.

When the heart rate identified by the analysis unit 40 satisfies a predetermined reference, the control unit 50 may control the stimulation providing type of the stimulation provided by at least one stimulation unit to be changed from a first stimulation providing type to a second stimulation providing type.

For example, a case in which the control unit 50 differently controls the stimulation providing type of the stimulation (vibration stimulation) provided by the vibration stimulation unit 61 as at least one stimulation unit in accordance with the identified heart rate is described below as an example. That is, a case in which the control unit 50 differently controls the stimulation providing type of the vibration stimulation unit 61 as an example in accordance with the heart rate is described below as an example.

As an example, when the identified heart rate is 100 times per minute, the control unit 50 may control the operation of the vibration stimulation unit 61 so as to generate vibrations of 100 times per minute (generate the vibration stimulation) in accordance with the heart rate with the strengths of the vibration of 5 stages among the strengths of the vibration divided into 5 stages (in this case, a first stage may be the slightest strength and a fifth stage may be the strongest strength). As another example, when the identified heart rate is 70 times per minute, the control unit 50 may control the operation of the vibration stimulation unit 61 so as to generate vibrations of 70 times per minute (generate the vibration stimulation) in accordance with the heart rate with the strengths of the vibration of second stages among the strengths of the vibration divided into 5 stages.

In an example of the present invention described above, controlling at least one stimulation unit in accordance with (in synchronization with) the heart rate is exemplified only as controlling at least one stimulation unit so that stimulations are made as large as the number of times corresponding to the same number as the heart rate, but is not limited thereto. As another example, in the present invention, controlling at least one stimulation unit in accordance with the heart rate may mean controlling at least one stimulation unit so that the stimulations are made as large as the number of times corresponding to the number of times set in proportion to the heart rate.

For example, it is assumed that the number of times set in proportion to the heart rate is 1/2. In this case, when the identified heart rate is 100 times per minute, the control unit 50 may control the operation of the vibration stimulation unit 61 so as to generate vibrations of 50 times (i.e., 50 times which is 1/2 of a total of 100 times) per minute (generate the vibration stimulation) in accordance with the heart rate with the strengths of the vibration of fifth stages among the strengths of the vibration divided into 5 stages. As another example, when the identified heart rate is 70 times per minute, the control unit 50 may control the operation of the vibration stimulation unit 61 so as to generate vibrations of 35 times (35 times which is 1/2 of 70 times) per minute (generate the vibration stimulation) in accordance with the heart rate with the strengths of the vibration of second stages among the strengths of the vibration divided into 5 stages.

As another example, a case in which the control unit 50 differently controls the stimulation providing type of the stimulation (odor stimulation) provided by the odor stimulation unit 64 as at least one stimulation unit in accordance with the identified heart rate is described below as an example. In this case, it is assumed that the number of times set in proportion to the heart rate is 1/50. Here, the number of times set in proportion to the heart rate may be set by a user input for each type of the stimulation (i.e., according to which stimulation among the vibration, sound, light, and odor stimulations the stimulation type is).

As an example, when the identified heart rate is 100 times per minute, the control unit 50 may control the operation of the odor stimulation unit 64 to generate the strengths of the odor (fragrance) of fifth stages (i.e., to generate the odor stimulation) two times (two times which is 1/50 of 100 times) per minute in accordance with the heart rate among the strengths of the odor (the discharge amount of the odor) divided into 5 stages (in this case, the first stage may mean a case where the strength is the slightest as the smallest amount of odor is discharged and the fifth stage may mean a case where the strength is the strongest as the largest amount of odor is discharged).

As such, the control unit 50 may differently control the stimulation providing type of at least one stimulation unit among the plurality of stimulation units 60 by considering the heart rate identified by the analysis unit 40 (in accordance with the heart rate).

Further, when the heart rate satisfies a predetermined reference, the control unit 50 may control the stimulation providing type of the stimulation provided by at least one stimulation unit to be changed from the first stimulation providing type to the second stimulation providing type. Here, the second stimulation providing type may mean a type in which the strength of the stimulation is strong, or the time of the stimulation is set to be long relatively as compared with the first stimulation providing type.

Further, the predetermined reference may mean a reference set to a predetermined threshold value or may mean a reference set to a plurality of predetermined range intervals.

For example, it is assumed that the predetermined reference as the reference set to the predetermined threshold value is set to '80 times or more per minute'.

When the heart rate of the user 3 analyzed by the analysis unit 40 is less than 80 times per minute, the control unit 50 may control the stimulation providing type of the vibration stimulation of the vibration stimulation unit 61 as at least one stimulation unit as the first stimulation providing type. Meanwhile, when the heart rate of the user 3 analyzed by the analysis unit 40 is equal to or more than 80 times (a predetermined threshold value) per minute, the control unit 50 may control the stimulation providing type of the vibration stimulation unit 61 to be changed from the first stimulation providing type to the second stimulation type.

Here, as an example, the first stimulation providing type is a type in which the vibration stimulation having the strength of the vibration of the second stage is generated for 20 seconds, and the second stimulation providing type may mean a type in which the vibration stimulation having the strength of the vibration of the fifth stage is generated for 40 seconds.

As another example, when the heart rate of the user 3 analyzed by the analysis unit 40 is less than 80 times per minute, the control unit 50 may control the stimulation providing type of the sound stimulation of the sound stimulation unit 62 as at least one stimulation unit as the first stimulation providing type. Meanwhile, when the heart rate of the user 3 analyzed by the analysis unit 40 is equal to or more than 80 times per minute, the control unit 50 may control the stimulation providing type of the sound stimulation unit 62 to be changed from the first stimulation providing type to the second stimulation type.

Here, as an example, the first stimulation providing type is a type in which the sound having the strength (magnitude) of the sound of the second stage is generated for 20 seconds, and the second stimulation providing type may mean a type in which the vibration stimulation having the strength of the sound of the fifth stage is generated for 40 seconds.

Further, for example, the predetermined reference is a reference set to a plurality of predetermined range intervals, and it is assumed that a first range interval is set to 'less than 70 times per minute', a second range interval is set to 'equal to or more 70 times and less than 80 times per minute', a third range interval is set to 'equal to or more than 80 times and less than 90 times per minute', and a fourth range interval is set to 'equal to more than 90 times per minute'.

In this case, when the heart rate belongs to a condition of the first range interval among a plurality of range intervals, the control unit 50 may control the stimulation providing type of the vibration stimulation of the vibration stimulation unit 61 as an example of at least one stimulation unit as the first stimulation providing type. Meanwhile, when the heart rate belongs to a condition of the second range interval, the control unit 50 may control the stimulation providing type of the vibration stimulation unit 61 as the second stimulation providing type and when the heart rate belongs to a condition of the third range interval, the control unit 50 may control the stimulation providing type of the vibration stimulation unit 61 as the third stimulation providing type. Further, when the heart rate belongs to a condition of the fourth range interval, the control unit 50 may control the stimulation providing type of the vibration stimulation unit 61 as the fourth stimulation providing type.

Here, as the first stimulation providing type proceeds to the fourth stimulation providing type, the strength of the stimulation may be set relatively stronger, and the time of the stimulation may be set longer.

As an example, when the heart rate belongs to the condition of the first range interval, the control unit 50 may control the vibration stimulation unit 61 to generate the strength of the vibration of the first stage for 10 seconds as the first stimulation providing type and when the heart rate belongs to the condition of the second range interval, the control unit 50 may control the vibration stimulation unit 61 to generate the strength of the vibration of the second stage for 20 seconds as the second stimulation providing type.

Further, when the heart rate belongs to the condition of the third range interval, the control unit 50 may control the vibration stimulation unit 61 to generate the strength of the vibration of the third stage for 30 seconds as the third stimulation providing type and when the heart rate belongs to the condition of the fourth range interval, the control unit 50 may control the vibration stimulation unit 61 to generate the strength of the vibration of the fourth stage for 40 seconds as the fourth stimulation providing type.

As such, by considering the reference set to the predetermined threshold value as the predetermined reference, when the heart rate of the user identified by the analysis unit 40 is equal to or more than the predetermined threshold value, the control unit 50 may control the stimulation providing type of the stimulation providing unit provided by at least one stimulation unit of the plurality of stimulation units 60 to be changed from the first stimulation providing type to the second stimulation providing type.

Further, by considering a reference related to the plurality of range intervals as the predetermined reference, when the heart rate identified by the analysis unit 40 is equal to or more than the predetermined threshold value, the control unit 50 may control the stimulation providing type of the stimulation providing unit provided by at least one stimulation unit of the plurality of stimulation units 60 to be changed differently for each of the plurality of range intervals according to a level of the heart rate of the user 3 analyzed by the analysis unit 40.

Meanwhile, the odor stimulation unit 64 may include a plurality of fragrance storage units storing a plurality of types of fragrance materials. Further, the odor stimulation unit 64 may include an injection nozzle unit connected to the plurality of fragrance storage units and injecting any one of fragrance materials stored in the plurality of fragrance storage units by the control of the control unit 50.

For example, it is assumed that the odor stimulation unit 64 includes three fragrance storage units as the plurality of fragrance storage units. In this case, the odor stimulation unit 64 may include a first fragrance storage unit storing a first fragrance material, a second fragrance storage unit storing a second fragrance material, and a third fragrance storage unit storing a third fragrance material. Further, the odor stimulation unit 64 may include an injection nozzle unit connected to the first fragrance storage unit, the second fragrance storage unit, and the third fragrance storage unit, and selectively injecting any one of the plurality of types of fragrance materials (e.g., the first fragrance material, the second fragrance material, and the third fragrance material).

In an example of the present invention, it is described that the injection nozzle unit is one component connected to all of the plurality of fragrance storage units, but is not limited only thereto, and the injection nozzle unit may be constituted by a plurality of injection nozzle units to be connected to the plurality of fragrance storage units, respectively. That is, as another example, the odor stimulation unit 64 may include a first injection nozzle unit connected to the first fragrance storage unit, a second injection nozzle unit connected to the second fragrance storage unit, and a third injection nozzle unit connected to the third fragrance storage unit.

In this case, the fragrance materials stored in the plurality of fragrance storage units, respectively may be types such as liquid, gas, solid, etc., but is not limited thereto.

Illustratively, the first fragrance material may be a lavender fragrance related material known to relieve stress, heart palpitations, headache, etc., and is effective for mental and physical stability, the second fragrance material may be a strawberry fragrance related material known to be excellent for relieving tension and anxiety and is effective for stabilizing emotions, and the third fragrance material may be a grapefruit fragrance related material known to have a good effect on the body and mind in various ways, such as relieving stress, removing depression, removing edema, removing migraine headache, and strengthening liver function. However, the example of such a fragrance is only an example for helping understanding of the present invention, and the present invention is not limited thereto, and materials related to various fragrances may be stored as the fragrance material stored in each of the plurality of fragrance storage units.

When the heart rate identified by the analysis unit 40 satisfies the predetermined reference, the control unit 50 may control the operation of the injection nozzle unit to inject the fragrance material stored in any one fragrance storage unit among the plurality of fragrance storage units. Meanwhile, when the heart rate identified by the analysis unit 40 does not satisfy the predetermined reference, the control unit 50 may control the operation of the injection nozzle unit to inject the fragrance material stored in any one fragrance storage unit other than the any one fragrance storage unit among the plurality of fragrance storage units.

As an example, when the heart rate identified satisfies the condition of 'equal to or more than 80 times per minute' as the predetermined reference, the control unit 50 may control the operation of the injection nozzle unit to inject the first fragrance material stored in the first fragrance storage unit as any one fragrance storage unit among the plurality of fragrance storage units. On the contrary, when the heart rate identified does not satisfy the condition of 'equal to or more than 80 times per minute' as the predetermined reference (i.e. in the case of less than 80 times per minute), the control unit 50 may control the operation of the injection nozzle unit to inject the second fragrance material stored in the second fragrance storage unit as any one fragrance storage unit other than the first fragrance storage unit among the plurality of fragrance storage units.

As such, the control unit 50 controls the injection nozzle unit to selectively inject any one fragrance material among the plurality of fragrance materials stored in the plurality of fragrance storage units according to the identified heart rate satisfying the predetermined reference (or according to the level of the heart rate) to change the fragrance provided to the user 3 according to a heart rate state of the user.

Further, the plurality of fragrance storage units in the odor stimulation unit 64 may be provided to be replaceable in the present device 100. Through a replacement, the present device 100 is enabled to be used for a longer period of time and convenience may be provided to the user.

Further, the odor stimulation unit 64 may include a sensing unit (not illustrated) sensing a remaining amount of the fragrance material stored in each of the plurality of fragrance storage units. Here, the sensing unit (not illustrated) may be, for example, at least one of a weight measurement sensor, a distance measurement sensor (or laser measurement sensor), and an image sensor, but is not limited thereto, and may adopt all types of sensors capable of measuring the remaining amount of the material. As an example, a plurality of sensing units (not illustrated) may be provided to correspond to the plurality of fragrance storage units, respectively.

When the remaining amount of the first fragrance material stored in the first fragrance storage unit among the plurality of fragrance storage units is equal to or less than a predetermined remaining amount as an analysis result of a sensing signal by a sensor unit (not illustrated), the control unit 50 may provide a replacement notification for replacing the first fragrance storage unit including the first fragrance material which is equal to or less than the predetermined remaining amount through at least one of the plurality of stimulation units 60. In this case, contents described for the first fragrance storage unit may also be equally applied to the description of each of the plurality of fragrance storage units in spite of contents omitted below.

For example, it is assumed that a liquid type of first fragrance material is stored in the first fragrance storage unit, a weight measurement sensor is provided in the first fragrance storage unit as the sensing unit (not illustrated), and the predetermined remaining amount is set to 10 g, for example.

In such a case, when a weight measurement value measured through the weight measurement sensor in the first storage unit is 10 g as an example, the control unit 50 may identify that the remaining amount of the first fragrance material is equal to or less than the predetermined remaining amount (i.e., 10 g), and as a result, the control unit 10 may provide the replacement notification for replacing the first fragrance storage unit through at least one stimulation unit among the plurality of stimulation units 60.

Illustratively, the control unit 50 may control the vibration stimulation unit 61 to turn on/off the vibration having the vibration strength of the fifth stage in a cycle of 1 second when providing the replacement notification through the vibration stimulation unit 61. As another example, the control unit 50 may control the operation of the sound stimulation unit 62 to provide a guide voice such as 'replacing the first fragrance material is required' when providing the replacement notification through the sound stimulation unit 62. Further, the control unit 50 may control the operation of the light stimulation unit 63 to maintain red light in an on state for 1 minute as an example when providing the replacement notification through the light stimulation unit 63.

As such, the control unit 50 checks the remaining amounts of the fragrance materials stored in the plurality of fragrance storage units based on the analysis result of the sensing signal by the sensor unit (not illustrated), and provides the replacement notification through at least one of the plurality of stimulation units 60 when the checked remaining amount is equal to or less than the predetermined remaining amount to allow the user to more easily and conveniently use or manage the present device 100.

The present invention proposes a real-time biofeedback device (the present invention, 100) using the ECG.

The present device 100 may monitor the heart state of the user in real time by using the ECG signal of the user, which is acquired (measured) through the electrocardiograph 10. Further, the present device 100 may feed back the ECG signal as at least one stimulation among the vibration, the sound, the light (color), and the odor to the user based on a result of analyzing the acquired ECG signal, which may guide the change in ECG signal of the user 3 and lead to a behavior change of the user. The present device 100 may be provided in a type such as a patch type, a watch type, etc., as described above.

The present device 100 may acquire the ECG signal of the user through the electrocardiograph 10 and determine the heart state (whether the heart state is the normal state, the abnormal suspicious state, the abnormal state, etc.) of the user by analyzing the acquired ECG signal (e.g., by an analysis using a medical algorithm) in real time, and record information on the determined heart state. That is, the present device 100 may store (record) the analysis result (i.e., the state diagnosis result) by the analysis unit 40 or information on the resulting operation control type of the plurality of stimulation units 60 in the database (not illustrated).

In other words, in the present device 100, the electrocardiograph 10 may be provided in the body of the user 3 in a form such as an electrode of a lead or attachment (i.e., the electrocardiograph may be attached to a part of the body of the user), and the ECG signal of the user 3 may be measured through the electrocardiograph 10. The present device 100 may determine the heart state (normal, abnormal suspicious, etc.) of the user in real time through the analysis of the ECG signal measured (acquired) through the electrocardiograph 10, or record information on the determined heart state.

The ECG measurement technology known in the related art is just used as a basic medical examination for diagnosing the health state of the user, and the technology for providing immediate feedback of the diagnosis result to the user may be insufficient.

Accordingly, the present device 100 may use a 'real-time ECG signal' acquired through the electrocardiograph 10 as an analysis source in order to stabilize or awaken the state of the user 3. The present device may acquire and collect the ECG signal of the user 3, which is measured by the electrocardiograph 10 implementable as various types (e.g., the patch type, the watch type, a handle, furniture, etc.) from the electrocardiograph 10. The present device 100 may feed back a current state of the user to the user through the plurality of stimulation units 60 (61, 62, 63, and 64) capable of providing various types of stimulations (stimulations such as the vibration, the sound, the light, the odor, etc.) embedded in the present device 100 based on the result (the state diagnosis result, etc.) of analyzing the ECG signal acquired by the electrocardiograph 10.

The present device 100 may stabilize or awaken the user through the feedback (i.e., by feeding back the heart state or the health state of the user with the vibration, the sound, the light, the odor, etc.). That is, through the feedback, the present device 100 may stabilize the state of the user and allow the user to awaken (recognize) a current heart state or health state thereof.

That is, the present device 100 may feed back at least one stimulation of the vibration, the sound, the light, and the odor to the user by controlling the operation of at least one stimulation unit among the plurality of stimulation units 60 including the vibration stimulation unit 61, the sound stimulation unit 62, the light stimulation unit 63, and the odor stimulation unit 63 in order to control the state (heart state or health state) of the user based on the analysis result (state diagnosis result) of the ECG signal. Through this, the present device 100 may stabilize the user and/or allow the user to awaken the state of the user.

When the present device 100 is provided to include the electrocardiograph 10, the present device 100 may be expressed differently as an electrocardiograph 10 integrated feedback device ((an electrocardiograph integrated biofeedback device), as an example. Further, when the present device 100 is provided as a component separated from the electrocardiograph 10, the present device 100 may be expressed differently as an electrocardiograph 10 separated feedback device (that is, an electrocardiograph separated biofeedback device), as an example.

The present device 100 feeds back at least one stimulation of the vibration, the sound, the light, and the odor according to the heart state of the user based on the analysis result of the ECG signal acquired through the electrocardiograph 10 to provide to enable the user to self-recognize the heart state or health state (whether the heart state or health state is the normal state, the abnormal state, the stable state, or the unstable state) thereof.

Illustratively, when the vibration stimulation is applied (biofeedback) to the body of the user 3 in accordance with the heart rate, a blood pressure may be lowered. According to this, the present device 100 provides feedback for at least one stimulation of the vibration, the sound, the light, and the odor in accordance with (in synchronization with) the heart rate identified by the analysis unit 40 to maximize mental and physical stability and awakens effects of the user.

Further, the present device 100 may analyze the state (the change in ECG signal) of the user before and after the use of a meditation app in conjunction with the meditation app as an example and control the operations of the plurality of stimulation units 60 based on the analysis result. As an example, when the present device 100 is applied to the car steering wheel, the present device may provide haptic feedback so that the driver may be awakened.

The present device 100 may provide at least one of the vibration, the sound, the light (light or color), and the odor as the plurality of types of stimulations according to the state of the user in order to stabilize the mind and body of the user and awaken the user.

The present device 100 may be provided in a type attachable or wearable onto a part of the body of the user and provided in a compact and light-weight type to be portable. When the present device is provided as the patch type, for example, the present device 100 may be expressed differently as a biofeedback patch type ECG terminal.

According to the present invention, it is possible to solve problems (i.e., the problems such as the limitation in the space, the complicated configuration due to the plurality of wires connected to the electrode, and the imitation of the movement/motion of the user) by providing the biofeedback device (the present device 100) using the electrocardiogram.

Further, the present invention may provide effects of reducing stress of the user, stabilizing the mind and body, and reducing the blood pressure through by providing the present device 100. The present device 100 may apply (biofeedback) the plurality of types of stimulations (vibration, sound, light and odor related stimulations) to the body of the user in accordance with the heart rate, and as a result, the blood pressure may be lowered.

Further, when the user performs meditation or yoga, for example, the present device 100 may allow the vibration stimulation unit 61 to apply the vibration stimulation to the user in synchronization with the heart rate of the user. The present device 100 may be applied to a yoga product, a massage chair, a car steering wheel, and the like, but is not limited thereto.

The sound stimulation provided through the sound stimulation unit 61 of the present device 100 is exemplified as being provided in the form of the guide voice (i.e., voice message) in the above-described example, but is not limited thereto, and the sound stimulation may be provided in the form of music (music by genre, such as ballad, R&B, music, and dance).

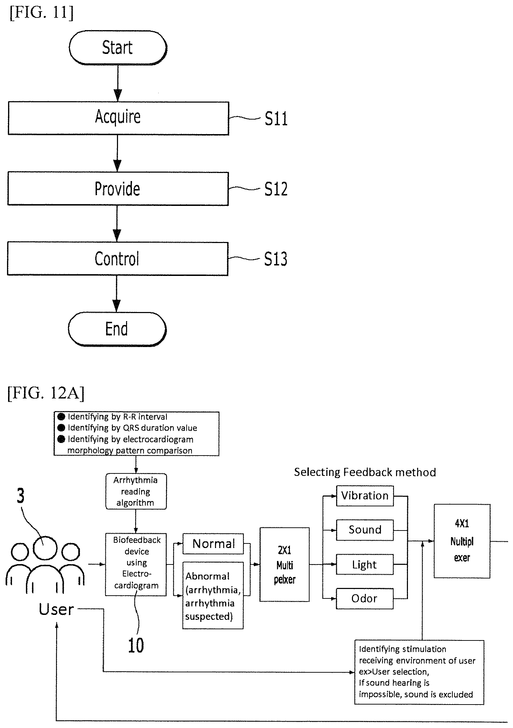

FIG. 12A is a diagram for describing a biofeedback principle by the biofeedback device 100 using the electrocardiogram according to an embodiment of the present invention.

Referring to FIG. 12A, the present device 100 may be provided as a type attached to a part of the body of the user 3, for example. The signal acquisition unit 30 of the present device 100 may acquire the ECG signal of the user 3 from the electrocardiograph 10 in the present device 100.

The analysis unit 40 may diagnose the state of the user through the analysis of the acquired ECG signal, and thus provide the state diagnosis result of the user.

The analysis unit 40 may diagnose (analyze) whether the heart state of the user 3 is the normal state or the abnormal state (e.g., an arrhythmia state, an arrhythmia suspected state, etc.) in relation to the state of the user.

In order to identify whether the user 3 is in the arrhythmia state, the analysis unit 40 may perform an analysis by applying an arrhythmia reading algorithm to the acquired ECG signal. Here, the arrhythmia reading algorithm may include an algorithm for identifying whether an arrhythmia is present at an R-R interval, an algorithm for identifying whether the arrhythmia is present based on a QRS duration value, and an algorithm for identifying an arrhythmia algorithm by comparing an electrocardiogram morphology pattern, but is not limited thereto, and may be applied to various arrhythmia reading algorithms known in the related art or developed in the future.

The analysis unit 40 may provide, as the state diagnosis result, whether the heart state of the user is normal or abnormal by using a 2×1 multiplexer, for example.

The control unit 50 may control the operation of at least one simulation unit among the plurality of simulation units 60 by using a 4×1 multiplexer, for example, based on the state diagnosis result provided by the analysis unit 40. The control unit 50 controls the operations of the plurality of stimulation units 60 to provide biofeedback related to at least one of the plurality of types of stimulations (e.g., the vibration stimulation, the sound stimulation, the light stimulation, and the odor stimulation) to the user 3.

In this case, when controlling the operation of at least one of the plurality of stimulation units 60 based on the state diagnosis result, the control unit 50 may control the operation of at least one stimulation unit in consideration of a stimulation receiving environment of the user, for example.

For example, it is assumed that based on information (e.g., information pre-selected for a case where the sound stimulation may not be received) pre-selected by the user 3, it is analyzed that there is an environment (i.e., when it is analyzed that an environment of the user is an environment in which it is impossible to hear the sound, for example, the inside of an airplane) in which the user may not receive the sound stimulation by the analysis unit 40. In this case, the control unit 50 may control the operation of at least one of the remaining stimulation units (i.e., the vibration stimulation unit, the light stimulation unit, and the odor stimulation unit) except for the sound stimulation unit 61 among the plurality of stimulation units 60 based on the information pre-selected by the user 3.

The present device 100 may allow the user 3 to maintain in the stable state or induce the user 3 to be awakened through controlling the operations of the plurality of stimulation units 60 by the control unit 50.

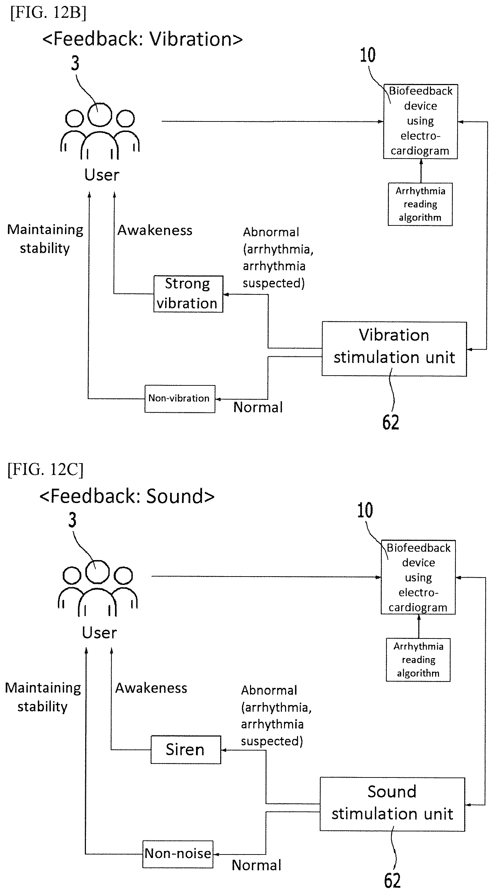

FIG. 12B is a diagram for describing a case of feeding back a vibration stimulation as a biofeedback in the biofeedback device 100 using the electrocardiogram according to an embodiment of the present invention.

Referring to FIG. 12B, the present device 100 may apply the arrhythmia reading algorithm to the acquired ECG signal to analyze the ECG signal of the user 3 acquired by the electrocardiograph 10. Through this, the control unit 50 of the present device 100 may control the operation of the vibration stimulation unit 61 as an example among the plurality of stimulation units 60 based on the state diagnosis result of the user 3. Through this, the control unit 50 may provide vibration (vibration stimulation) related biofeedback to the user through the control of the vibration stimulation unit 61.

In this case, when it is analyzed that the heart state of the user is the normal state as the state diagnosis result, the control unit 50 may control the vibration simulation unit 61 so that the vibration simulation unit 61 does not vibrate (i.e., so that the vibration stimulation unit 61 operates without vibration). The control unit 50 may maintain the state of the user 3 in the stable state by controlling the vibration stimulation unit 61 without vibration.

Meanwhile, when the heart state of the user is abnormal (in the case of the arrhythmia state or the arrhythmia suspected state) as the state diagnosis result, the control unit 50 may control the vibration stimulation unit 61 to provide the vibration with the vibration strength of the fifth stage which is the vibration having the strongest strength (intensity) among the strengths of the vibration divided into 5 stages. The control unit 50 may provide (induce) the user 3 to awaken (recognize) that the heart state of the user is the abnormal state (in particular, the arrhythmia state or the arrhythmia suspected state) by providing the vibration stimulation having the vibration strength of the fifth stage as an example through the vibration simulation unit 61.

FIG. 12C is a diagram for describing a case of feeding back a sound stimulation as the biofeedback in the biofeedback device 100 using the electrocardiogram according to an embodiment of the present invention.

Referring to FIG. 12C, the present device 100 may apply the arrhythmia reading algorithm to the acquired ECG signal. Through this, the control unit 50 of the present device 100 may control the operation of the sound stimulation unit 62 as an example among the plurality of stimulation units 60 based on the state diagnosis result of the user 3. Through this, the control unit 50 may provide sound (sound stimulation) related biofeedback to the user through the control of the sound stimulation unit 62.

In this case, when it is analyzed that the heart state of the user is the normal state as the state diagnosis result, the control unit 50 may control the sound simulation unit 62 so that the sound simulation unit 62 does not generate the sound (i.e., so that the sound simulation unit 62 operates without sound). The control unit 50 may maintain the state of the user 3 in the stable state by controlling the sound stimulation unit 62 without sound.

Meanwhile, when the heart state of the user is abnormal (in the case of the arrhythmia state or the arrhythmia suspected state) as the state diagnosis result, the control unit 50 may control the sound stimulation unit 62 to provide the sound with a largest strength among the strengths of the sound divided into 5 stages (or to provide a siren sound). The control unit 50 may provide (induce) the user 3 to awaken (recognize) that the heart state of the user is the abnormal state (in particular, the arrhythmia state or the arrhythmia suspected state) by providing the sound stimulation related to the siren sound as an example through the sound simulation unit 62.

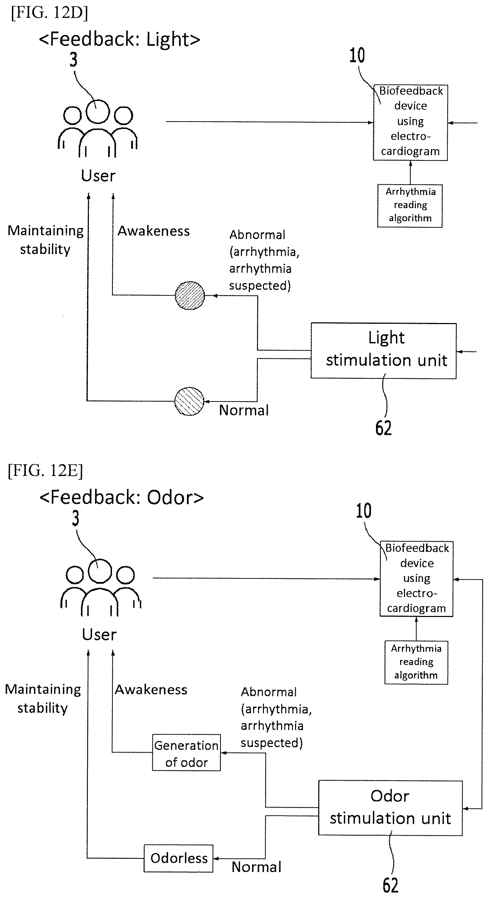

FIG. 12D is a diagram for describing a case of feeding back a light stimulation as the biofeedback in the biofeedback device 100 using the electrocardiogram according to an embodiment of the present invention.

Referring to FIG. 12D, the present device 100 may apply the arrhythmia reading algorithm to the acquired ECG signal. Through this, the control unit 50 of the present device 100 may control the operation of the light stimulation unit 63 as an example among the plurality of stimulation units 60 based on the state diagnosis result of the user 3. Through this, the control unit 50 may provide light (light stimulation) related biofeedback to the user through the control of the light stimulation unit 63.

In this case, when it is analyzed that the heart state of the user is the normal state as the state diagnosis result, the control unit 50 may control the light simulation unit 63 so that green light is emitted from the light simulation unit 63 as an example. The control unit 50 may maintain the state of the user 3 in the stable state by controlling the light stimulation unit 62 to emit the green light.

Meanwhile, when it is analyzed that the heart state of the user is abnormal (in the case of the arrhythmia state or the arrhythmia suspected state) as the state diagnosis result, the control unit 50 may control the light simulation unit 63 so that red light is emitted from the light simulation unit 63 as an example. The control unit 50 may provide (induce) the user 3 to awaken (recognize) that the heart state of the user is the abnormal state (in particular, the arrhythmia state or the arrhythmia suspected state) by controlling the light stimulation unit 62 to emit the red light.

FIG. 12E is a diagram for describing a case of feeding back an odor stimulation as the biofeedback in the biofeedback device using the electrocardiogram according to an embodiment of the present invention.

Referring to FIG. 12E, the present device 100 may apply the arrhythmia reading algorithm to the acquired ECG signal. Through this, the control unit 50 of the present device 100 may control the operation of the odor stimulation unit 64 as an example among the plurality of stimulation units 60 based on the state diagnosis result of the user 3. Through this, the control unit 50 may provide sound (sound stimulation) related biofeedback to the user through the control of the odor stimulation unit 64.

In this case, when it is analyzed that the heart state of the user is the normal as the state diagnosis result, the control unit 50 may control the odor simulation unit 64 so that no odor is discharged from the odor simulation unit 64 (i.e., so that there is odorless) as an example. The control unit 50 may maintain the state of the user 3 in the stable state by controlling the odor stimulation unit 64 to operate without odor.

Meanwhile, when it is analyzed that the heart state of the user is abnormal (in the case of the arrhythmia state or the arrhythmia suspected state) as the state diagnosis result, the control unit 50 may control the operation of the odor simulation unit 64 so that the odor is generated from the odor simulation unit 64 as an example. In this case, the control unit 50 may control the odor stimulation unit 64 so that fragrance (odor) corresponding to any one fragrance material among a plurality of types of fragrance materials is discharged. The control unit 50 may provide (induce) the user 3 to awaken (recognize) that the heart state of the user is the abnormal state (in particular, the arrhythmia state or the arrhythmia suspected state) by controlling the odor sound stimulation unit 64 to generate the odor (to discharge the odor).

Hereinafter, a technology (i.e., an ECG lead guide technology) which guides the user 3 to attach the electrocardiograph 10 in an intended electrocardiogram lead direction by detecting an attachment direction (position, angle, etc.) of the electrocardiograph 10 using a motion sensor embedded in the wearable or attachable electrocardiograph 10 without a separate lead line and transmitting the detected information to the user terminal 20 (as a mobile terminal, smartphone, tablet, PC, etc.) wirelessly (Bluetooth, Wi-Fi, etc.) will be described.

Hereinafter, in describing the ECG lead guide technology, the contents described for the present system 1000, the present device 100, the electrocardiograph 10, the user terminal 20, etc., may also be equally applied to the description of the ECG lead guide technology in spite of the contents omitted below.

Further, in the following description, in describing the ECG lead guide technology, for example, it will be exemplified that the electrocardiograph 10 is an electrocardiograph (the electrocardiograph included in the present device) provided integrally with the present device 100 as an example. However, the present invention is not limited only thereto, and as described above, as another example, the electrocardiograph 10 may also be provided as a separate component separated from the present device 100.

In relation to the ECG lead guide technology, referring back to FIG. 2, the electrocardiograph 10 included in the present device 100 may be attached to a part of the body of the user 3. In other words, the present device 100 including the electrocardiograph 10 may be attached to a part of the body of the user 3. In the following description, the user 3 may be expressed differently as the patient.

The electrocardiograph 10 may acquire posture information and the ECG signal by using a plurality of sensors. As an example, the electrocardiograph 10 may be a terminal, a patch type, or a wearable product. Further, the posture information may include position and angle information of the electrocardiograph 10. The electrocardiograph 10 may be attached in a specific electrocardiogram lead direction to acquire the ECG signal. Further, the electrocardiograph 10 may include a sensor unit 11 for acquiring the posture information and an electrode (sensor) for acquiring the ECG signal. For example, the posture information may include information related to the current position and direction of the electrocardiograph 10.

In order to determine the user's accurate heart state, the electrocardiograph 10 is required to be attached to a part (e.g., near the heart) of the body of the user 3 according to a predetermined direction (i.e., the direction of reference posture information to be described later). That is, the electrocardiograph 10 should be attached to the position and direction of a predetermined electrode to acquire an accurate waveform of the electrocardiogram.

By the electrocardiograph 10, the electrocardiogram in the range of several mV measured around the heart may be filtered through a low pass filter (LPF) and applied as an input of an instrumentation amplifier (IA) through a buffer. At the IA stage, an electrocardiogram waveform may be output by amplifying 1000 times, and electrocardiogram signals in the range of 1 Hz to 35 Hz by a band pass filter (BPF) which is a combination of the LPF connected with the IA stage output and a high pass filter (HPF). In addition, the electrocardiograph 10 may filter analog ECG signals by hardware capable of measuring ECG signals and driving the hardware and convert the analog ECG signals into digital signals to transmit the converted signals to the user terminal 20. In addition, the electrocardiograph 10 may transmit only electrocardiogram signals converted into digital signals through an analog-to-digital converter to the user terminal 20. For example, the analog-to-digital converter may have 24-bit resolution and a sampling frequency of 250 Hz. However, the configuration of the electrocardiograph 10 is not limited thereto.

The user terminal 20 may provide guide information for guiding the attachment direction of the electrocardiograph 10 based on the posture information acquired from the electrocardiograph 10. The guide information may be used to provide information on the attachment direction of the electrocardiograph 10 to be attached to acquire existing standardized electrocardiogram signals.

In other words, in relation to the electrocardiograph 10 provided integrally with the present device 100 or the electrocardiograph 10 provided as the component separated from the present device 10, the user terminal 20 may provide guide information (feedback information) for guiding so that the attachment direction of the electrocardiograph 10 is attached to the body part of the user 3 may be attached to be more precisely matched to the direction of the user's intended electrocardiogram lead.

By the guide information provided through the user terminal 20, the attachment direction of the electrocardiograph 10 may be adjusted.

In this case, when the present device is the electrocardiograph 10 integrated biofeedback device, the user may adjust the attachment direction of the present device 100 itself including the electrocardiograph 10. As another example, when the present device is the electrocardiograph 10 integrated biofeedback device, the control unit 50 of the present device 100 may automatically adjust the attachment direction of the electrocardiograph 10 embedded in the present device 100 based on the guide information provided from the user terminal 20. As yet another example, when the present device is the electrocardiograph 10 separated biofeedback device, the user may adjust the attachment direction of the electrocardiograph 10 by directly moving the electrocardiograph 10.

For example, the user terminal 20 may receive user input information for measuring an electrocardiogram in a lead 1 direction. The user terminal 20 may provide guide information so that the electrocardiograph 10 is positioned on the left chest (above the nipple) of the user 3. In addition, in order to measure the electrocardiogram in the lead 1 direction, the user terminal 20 may provide guide information so that a positive (+) electrode provided in the electrocardiograph 10 is positioned close to a left arm LA of the user 3 and a negative (-) electrode is positioned close to a right arm RA of the user 3. In addition, the electrocardiograph 10 may estimate an angle difference between the first direction including preset reference information of lead 1 and direction information acquired based on a sensor. The user terminal 20 may provide different output characteristics of the guide information based on the angle difference information estimated from the electrocardiograph 10.

Illustratively, for electrocardiogram lead, medically and academically, measurement directions Lead I, II, III, aVL, aVR, aVF, V1, V2, V3, V4, V5, and V6 have already been standardized. Standard 12-lead electrocardiograms are divided into bipolar leads, which record a potential difference between two parts, and unipolar leads, which record a potential at a part to which the electrode is attached. The standard bipolar leads may be divided into Lead I, II, and III. Lead I means a voltage between a left arm (LA) electrode and a right arm (RA) electrode. (I = LA - RA) lead II means a voltage between a left leg (LL) electrode and a right arm (RA) electrode. (II = LL - RA) lead III means a voltage between a left leg (LL) electrode and a left arm (LA) electrode.

In addition, unipolar limb leads are divided into aVL, aVR, and aVF, and electrodes are connected to the right hand, left hand, and left foot to record an electrocardiogram. However, since the electrocardiogram waveform recorded by the unipolar limb leads is small in size, aVR, aVL, and aVF amplified with the electrocardiogram waveform by 1.5 times may be used. Here, a may mean the first letter of augmented.

Precordial leads are divided into V1, V2, V3, V4, V5, and V6, and the standard leads or the limb leads have a disadvantage of recording the electrocardiogram at a region far away from the heart, but the precordial leads only record the electrocardiogram at a region closer to the heart. The position of each precordial lead, V1 may be located right next to the right sternum in the fourth intercostal space (between ribs 4 and 5). V2 may be located right next to the left sternum in the fourth intercostal space (between ribs 4 and 5). V3 may be located in the middle region between V2 and V4. V4 may be located at the midclavicular line of the fifth intercostal space (between ribs 5 and 6). V5 may be located at the left of the anterior armpit horizontally in parallel with V4. V6 may be located on the midaxillary line horizontally in parallel with V4 and V5.

As an example, the user terminal 20 may output an electrocardiogram lead guide menu, an electrocardiogram measurement menu, an electrocardiogram attachment guide menu, and the like. For example, an application program for providing the electrocardiogram lead guide menu and the electrocardiogram measurement menu is installed in the user terminal 20, and the electrocardiogram lead guide menu, the electrocardiogram measurement menu, and the electrocardiogram attachment guide menu may be provided.

FIG. 6 is a schematic block diagram of the electrocardiograph 10 included in the biofeedback device 100 using the electrocardiogram according to an embodiment of the present invention.

The electrocardiograph 10 included in the present device 100 as the electrocardiograph 10 according to an embodiment of the present invention and will be hereinafter referred to as the present electrocardiograph 10 for convenience of description.

Referring to FIG. 6, the present electrocardiograph 10 may include a sensor unit 11, a generation unit 12, a detection unit 13, and a communication unit 14. However, the configuration of the electrocardiograph 10 is not limited thereto. For example, the electrocardiograph 10 may include a power supply unit (not illustrated) for applying power.

The sensor unit 11 may acquire position and angle information using a plurality of sensors. In other words, the sensor unit 11 may acquire the position and angle information of the electrocardiograph 10 using an acceleration sensor and a gyro sensor. For example, the plurality of sensors may be an acceleration sensor and a gyro sensor. The sensor unit 11 may acquire the position and angle information of the electrocardiograph 10 using the acceleration sensor and the gyro sensor. In addition, the sensor unit 11 may acquire current position and angle information of the electrocardiograph 10 including information of an X coordinate, a Y coordinate, a Z coordinate, and the like using the plurality of sensors. In addition, the sensor unit 11 may acquire current position and angle information including information of a roll, a pitch, a yaw, and the like.

For example, the acceleration sensor is a sensor that measures a dynamic force such as acceleration, vibration, impact, or the like of an object, and refers to a sensor capable of detecting a motion state of an object. The gyro sensor measures angular velocity, unlike an acceleration sensor that measures acceleration. As another example, the plurality of sensors of the sensor unit 11 may include an accelerometer, an angular velocimeter, a geomagnetic meter, a GPS, a computer vision, and the like.

In addition, the sensor unit 11 may include electrodes (sensors). The sensor unit 11 may acquire electrocardiogram signals of the user 3 from the electrodes (sensors). The sensor unit 11 may acquire ECG signals. The ECG signals are called electrocardiogram or electrocardiogram signals and refer to those recorded in the form of wavelengths by analyzing the electrical activity of the heart. More specifically, the ECG signals are waveforms consisting of a vector sum of action potentials generated by the special excitatory & conductive system of the heart. That is, the ECG signals are signals that measure, from electrodes contacted outside the body, vector sum signals of the action potentials generated from the sinoatrial node (SA node), the atrioventricular node (AV node), the His bundle, the bundle branch, purkinje fibers, and the like, which are components of the heart. When measuring the electrocardiogram, two or more electrodes are used, and the electrodes are paired.

Although the above-mentioned ECG signals, the electrocardiogram or the electrocardiogram signals are used interchangeably, they are not different from each other and may mean the same electrocardiogram signal.

The generation unit 12 may predict current posture information of the electrocardiogram based on the position and angle information of the electrocardiogram acquired from the sensor unit 11. For example, the generation unit 12 may calculate a current position and a current posture of the electrocardiograph 10. The generation unit 12 may predict the current position of the electrocardiograph 10 including information on an X coordinate, a Y coordinate, a Z coordinate, and the like, and predict the current posture of the electrocardiograph 10 including information on a roll, a pitch, a yaw, and the like. The roll refers to rotation in front and rear axes, the pitch refers to up and down movement of the electrocardiograph 10, and the yaw refers to an angle tilted to one side. The generation unit 12 may generate direction information by calculating a current position and a current posture (angle) of the electrocardiograph 10.

In addition, the generation unit 12 calculates an angle difference between reference posture information and current posture information to estimate a difference between the reference posture information and the current posture information. The reference posture information may be set in response to each of a plurality of pieces of reference direction information. In addition, the generation unit 12 may estimate an angle difference between preset first reference posture information and the current posture information. In addition, the generation unit 12 may estimate an angle difference between preset second reference posture information and the current posture information. In addition, the generation unit 12 may estimate an angle difference between preset third reference posture information and the current posture information. Here, the first reference posture information may be information associated with lead I. In addition, the second reference posture information may be information associated with lead II. In addition, the third reference posture information may be information associated with lead III.

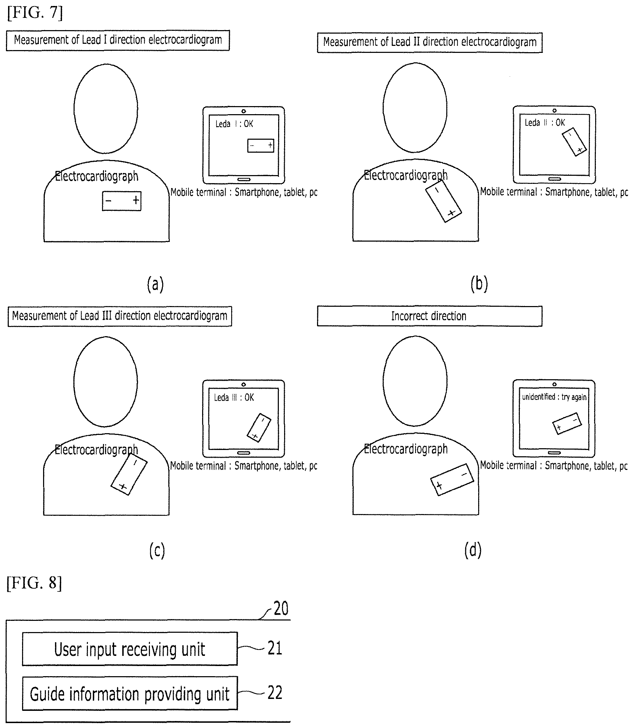

FIG. 7 is a diagram schematically illustrating an attachment direction of the electrocardiograph 10 included in the biofeedback device 100 using the electrocardiogram according to an embodiment of the present invention. That is, FIG. 7 is a diagram illustrating an example of the attachment direction of the electrocardiograph 10 in order to provide the ECG lead guide technology in relation to the electrocardiograph 10 included in the present device 100.

With reference to the reference posture information, illustratively, referring to FIG. 7A, Lead I is for recording a potential difference between a left-hand electrode (+) and a right hand electrode (-), and needs to be located so that the - and + electrodes of the electrocardiograph 10 are horizontal with each other. In other words, the reference posture information of Lead I may be a case in which the - electrode and the + electrode are in a horizontal state.

In addition, referring to FIG. 7B, Lead II is for recording a potential difference between a left foot electrode (+) and a right-hand electrode (-), and may be tilled at a specific angle (e.g., -44°) based on the + electrode of the electrocardiograph 10 to acquire electrocardiogram signals. In other words, the reference posture information of Lead II may be a case in which the - electrode and the + electrode are tilted at a specific angle. For example, in the electrocardiograph 10, in order to measure the electrocardiogram in the direction of lead II, the - electrode may be positioned to be tilted in the right-hand direction and the + electrode may be positioned to be tilted in the left-foot direction.

In addition, referring to FIG. 7C, Lead III is for recording a potential difference between a left foot electrode (+) and a left foot electrode (-), and may be tilled at a specific angle (e.g., -134°) based on the + electrode of the electrocardiograph 10 to acquire electrocardiogram signals. In other words, the reference posture information of Lead II may be a case in which the - electrode and the + electrode are tilted at a specific angle. For example, in the electrocardiograph 10, in order to measure the electrocardiogram in the direction of lead III, the - electrode may be positioned to be tilted in the left-hand direction and the + electrode may be positioned to be tilted in the left-foot direction.

In addition, the generation unit 12 may estimate an angle difference between preset reference posture information and the current posture information. Illustratively, referring to FIG. 7, a blue line illustrated in FIGS. 7A and 7B may be referred to as the reference posture information of lead I, and a red line may be referred to as the current posture information.

When the reference posture information and the current posture information of Lead I match each other, the generation unit 12 may generate angle information of the current posture information of the electrocardiograph 10 as 0°. In addition, when a difference between the reference posture information and the current posture information of Lead I is 23° as illustrated in FIG. 7A, the generation unit 12 may generate angle information of the current posture information of the electrocardiograph 10 as 23°. In addition, when a difference between the reference posture information and the current posture information of Lead I is -8° as illustrated in FIG. 7A, the generation unit 12 may generate angle information of the current posture information of the electrocardiograph 10 as -8°.

The detection unit 13 may detect whether the electrocardiograph 10 is attached to a part of the body of the user 3. For example, the detection unit 13 may detect whether the electrode (sensor) has been attached to the body part (e.g., above the left chest nipple) of the user 3. The detection unit 13 may detect the attachment based on a temperature, an electrocardiogram acquisition signal, etc., but is not limited thereto.

The communication unit 14 may transmit current posture information and electrocardiogram lead signals to the user terminal 20. In addition, the communication unit 14 may transmit an angle difference between preset reference posture information and current posture information to the user terminal 20. For example, the communication unit 15 may transmit the information and signals acquired from the electrocardiograph 10 to the user terminal 20 via a network. The communication unit 15 may transmit direction information and electrocardiogram lead signals to the user terminal 20 via a network such as Bluetooth or Wi-Fi.

Further, when the attachment detected by the detection unit 13 is On, the communication unit 14 may provide (transmit), to the user terminal 20, the angle difference information between the preset reference posture information and the current posture information estimated by the generation unit 12, and the ECG signal. When the attachment detected by the detection unit 13 is On, the electrode (sensor) may acquire the electrocardiogram signals, and when the attachment detected by the detection unit 13 is On, the communication unit 14 may provide (transmit), to the user terminal 20, the angle difference information estimated by the generation unit 12 and the electrocardiogram signals acquired from the sensor unit 11.

On the contrary, when the attachment detected by the detection unit 13 is Off, the communication unit 14 may provide (transmit), to the user terminal 20, the angle difference information between the preset reference posture information and the current posture information estimated by the generation unit 12.

FIG. 8 is a block diagram illustrating a schematic configuration of a user terminal 20 included in the biofeedback system 1000 using the electrocardiogram according to an embodiment of the present invention. In particular, FIG. 8 is a diagram for describing a function of the user terminal 20 required to provide the ECG lead guide technology.

Referring to FIG. 8, the user terminal 20 may include a user input receiving unit 21 and a guide information providing unit 22 in order to provide the ECG lead guide technology. However, the configuration of the user terminal 20 is not limited thereto. For example, the user terminal 20 may include a data storage unit (not illustrated) for storing information received from the electrocardiograph 10.

The user input receiving unit 21 may receive user input information for selecting at least one of a plurality of reference direction information items. For example, the plurality of reference direction information items may include a Lead I direction information item, a Lead II direction information item, and a Lead III direction information item.

The user may select any one of electrocardiogram lead direction items to be acquired from among the plurality of reference direction information items. For example, the user input receiving unit 21 may receive user input information corresponding to the Lead I direction information item. The guide information providing unit 22 may provide guide information associated with position and direction information to be attached with the electrocardiograph 10 in order to acquire a Lead I direction electrocardiogram measurement signal in response to the Lead I direction information item that is the user input information.

The guide information providing unit 22 may provide guide information based on the user input information and the angle difference information provided from the electrocardiograph. In other words, the guide information providing unit 22 may provide guide information of guiding the attachment direction of the electrocardiograph 10 based on the user input information and the angle difference information between the preset reference posture information and the current posture information provided from the electrocardiograph 10.

For example, the guide information providing unit 22 may provide guide information associated with position and direction information to be attached with the electrocardiograph 10 in order to acquire a Lead I direction electrocardiogram measurement signal in response to the Lead I direction information item that is the user input information.

In addition, the guide information providing unit 22 may provide guide information so that the current posture information and the preset reference posture information match each other, based on the angle difference information provided from the electrocardiograph 10.

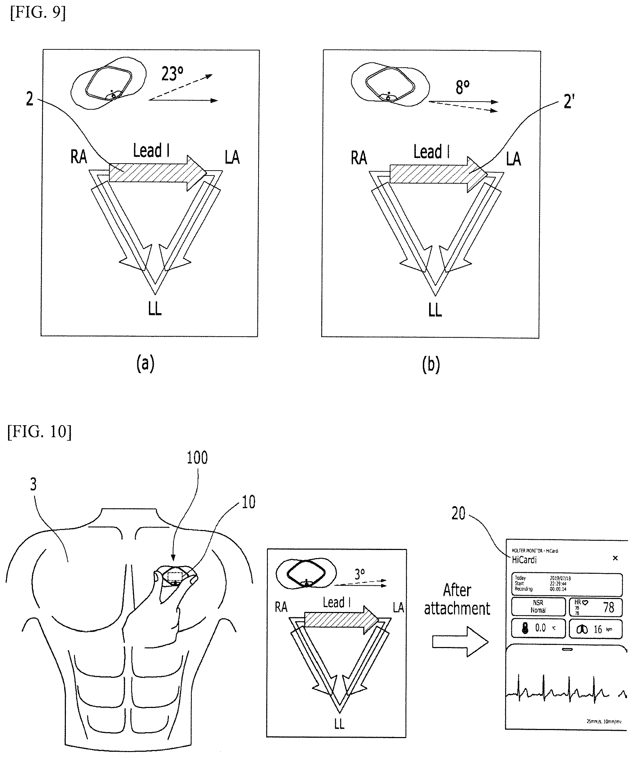

FIG. 9 is a diagram illustrating an example in which a guide information providing unit of the user terminal 20 included in the biofeedback system 1000 using the electrocardiogram according to an embodiment of the present invention outputs guide information.

Referring to FIG. 9, the guide information providing unit 22 may vary output characteristics of the guide information based on the user input information and the angle difference information. As an example, the output characteristics may include at least one of blinking speed, vibration, and sound. In order words, the guide information providing unit 22 may vary output characteristics of the guide information based on the attachment direction of the electrocardiograph 10 associated with the reference posture information corresponding to the user input information and the angle difference information between the preset reference posture information provided from the electrocardiograph 10 and the current posture information.

The guide information providing unit 22 may change the blinking speed based on the angle difference corresponding to reference information of a preset angle. For example, when the angle difference information is ± 60° or more, the guide information providing unit 22 may vary the output characteristics by blinking at a cycle of 2 seconds. In addition, in the case of ± 30° or more and less than ± 60°, the guide information providing unit 22 may vary the output characteristics by blinking at a cycle of 1.5 second. In addition, in the case of ± 10° or more and less than ± 30°, the guide information providing unit 22 may vary the output characteristics by blinking at a cycle of 1.0 second. In addition, in the case of ± 5° or more and less than ± 10°, the guide information providing unit 22 may vary the output characteristics by blinking at a cycle of 0.5 second. In addition, in the case of less than ± 5 °, the guide information providing unit 22 may vary the output characteristics by stopping red blinking and displaying blue.

Illustratively, referring to FIG. 9A, the guide information providing unit 22 may allow a first arrow 2 to be output in red when the user input receiving unit 21 receives the user input information corresponding to the Lead I direction information item. In addition, the guide information providing unit 22 may change output characteristics of the first arrow 2 based on the angle difference information, that is, the angle difference information between the preset first reference posture information and the current posture information.

The guide information providing unit 22 may receive the angle difference information of 23° from the electrocardiograph 10 and change the output characteristics of the first arrow 2 based on the output characteristics corresponding to the preset angle difference information. In the case of FIG. 9A, since the angle difference information is 23°, the guide information providing unit 22 may change the output characteristics so that the first arrow 2 blinks at a cycle of 1.0 second.

Further, referring to FIG. 9B, the guide information providing unit 22 may allow a second arrow 2' to be output in red when the user input receiving unit 21 receives the user input information corresponding to the Lead I direction information item. In addition, the guide information providing unit 22 may change the output characteristics of the second arrow 2' based on the angle difference information, that is, the angle difference information between the preset second reference posture information and the current posture information.

The guide information providing unit 22 may receive the angle difference information of -8° from the electrocardiograph 10 and change the output characteristics of the second arrow 2' based on the output characteristics corresponding to the preset angle difference information. In the case of FIG. 9B, since the angle difference information is -8°, the guide information providing unit 22 may change the output characteristics so that the second arrow 2' blinks at a cycle of 0.5 second.

FIG. 10 is a diagram for describing an electrocardiogram lead guide technology using the electrocardiograph 10 and the user terminal 20 included in the biofeedback system 1000 using the electrocardiogram according to an embodiment of the present invention.

Referring to FIG. 10, according to an embodiment of the present invention, a user may prepare the electrocardiograph 10 and the user terminal 20 provided in the present invention to receive an electrocardiogram lead guide technology.

The electrode (sensor) may be attached to and provided in the electrocardiograph 10 and the user may turn on the electrocardiograph 10. In addition, the user may receive an electrocardiogram lead guide technology by executing an electrocardiogram lead guide application downloaded to the user terminal 20.

The user may select at least one of Leads I, II, and III to be measured in the electrocardiogram lead guide application downloaded to the user terminal 20. The user terminal 20 may vary an output of any one of arrows of a lead selected in a triangle based on user input information.

The user may position an electrode attachment surface of the electrocardiograph 10 on the left chest (above the nipple) toward the body surface and without being attached to the body surface, for example. The electrocardiograph 10 may predict current posture information by using the sensor unit 11. In addition, the electrocardiograph 10 may estimate an angle difference between the preset reference posture information and the current posture information and transmit the estimated angle difference information to the user terminal 20.

The user terminal 20 may display a difference between the direction of the electrocardiograph 10 and the input information (information of the selected lead) of the user with an angle and an arrow on a screen of the user terminal 20. In addition, the user terminal 20 may output differently the blinking speed of the arrow on the screen based on the estimated angle difference information.

The user may adjust the position of the electrocardiograph 10 so that the angle difference information displayed on the user terminal 20 is within 5°, and attach the electrocardiograph 10 to the body part of the user 3 when the angle difference information displayed on the user terminal 20 is within 5° and then the blinking of the letter of the arrow stops.

The user terminal 20 may output electrocardiogram signals and heart rate information acquired from the electrocardiograph 10 when the electrocardiograph 10 is attached to the body part of the user 3.

In general, an electrocardiograph is a device that detects and amplifies the electromotive force generated by cardiac muscle activity as a potential difference between two points on the body surface and then displays the electromotive force as a waveform on a recording paper. In general, the electrocardiograph is used to diagnose heart disease by analyzing the minute waveform that records the potential difference generated in the heart and both arms and legs, which are the extremities of the body. The electrocardiograph includes electrocardiographs used in hospitals and medical institutions, portable electrocardiographs that may be used at home or in daily life, and wearable electrocardiographs, and the like, and recently, an electrocardiograph that is connected a smartphone and measurable has also been developed.

For electrocardiogram lead, medically and academically, measurement directions Lead I, II, III, aVL, aVR, aVF, V1, V2, V3, V4, V5, and V6 have already been standardized. In a wearable or attachable electrocardiograph without a separate lead line having a degree of freedom, the electrocardiogram lead is measured differently depending on an attachment direction of the electrocardiograph. When the wearable or attachable electrocardiograph is attached, there is a problem in that it is difficult to obtain an intended electrocardiogram lead signal if a standard lead direction is not accurately aligned. Current products are fixed in a specific direction to measure the electrocardiogram.

Meanwhile, there are cases in which the electrocardiogram lead needs to be applied differently depending on the patient's symptoms and specifics. In this case, when a non-medical person who is not a skilled medical person self-wears the electrocardiograph, there is a problem in that accurate electrocardiogram signals may not be obtained because the electrocardiograph is attached in an incorrect direction.

Therefore, according to the ECG lead guide technology proposed in the present invention, the present system 1000 may guide a user to attach an electrocardiograph in an intended electrocardiogram lead direction by detecting an attachment direction (position, angle, etc.) of the electrocardiograph using a (motion) sensor embedded in the electrocardiograph and transmitting the detected attachment direction to a mobile terminal (smartphone, tablet, PC) by wireless (Bluetooth, Wi-Fi, etc.).

That is, according to the ECG lead guide technology provided by the electrocardiograph 10 and the user terminal 20 in the present system 1000, the present invention may guide a user to attach an electrocardiograph in an intended electrocardiogram lead direction by detecting an attachment direction (position, angle, etc.) of the electrocardiograph using a (motion) sensor embedded in the electrocardiograph and transmitting the detected attachment direction to a mobile terminal (smartphone, tablet, PC) by wireless (Bluetooth, Wi-Fi, etc.) to allow a non-medical person who is not a skilled medical person to attach the electrocardiograph in an accurate direction.

Hereinafter, an operational flow of the present invention will be briefly described based on the contents described above in detail.

FIG. 11 is an operation flowchart for a control method of a biofeedback device using an electrocardiogram according to an embodiment of the present invention.

The control method of the biofeedback device using the ECG illustrated in IF. 11 may be performed by the present device 100 described above. Therefore, in spite of the contents omitted below, the contents described for the present device 100 may also be equally applied to the description of the control method of the biofeedback device using the ECG.

Referring to FIG. 11, in step S11, a signal acquisition unit may acquire an ECG signal of a user from an electrocardiograph.

Next, in step S12, an analysis unit may analyze the ECG signal acquired in step S11 and provide a state diagnosis result of the user to a control unit as an analysis result.

Further, in step S12, when there is a waveform which satisfies a predetermined feature condition within a waveform of the ECG signal acquired in step S11, the analysis unit may identify that a signal corresponding to the waveform satisfying the predetermined feature condition is an abnormal suspicious ECG signal.

Further, in step S12, the analysis unit finally identifies whether the abnormal suspicious ECG signal is an abnormal ECG signal based on a comparison of a similarity between waveform data of a plurality of abnormal ECG signals prestored in a database and waveform data of the abnormal suspicious ECG signal to provide the state diagnosis result.

Further, in step S12, the analysis unit may identify a heart rate of the user by analyzing the ECG signal.

Next, in step S13, the control unit may control at least one simulation unit among a plurality of simulation units for the biofeedback for state control of the user based on the state diagnosis result in step S12.

In this case, according to step S13, a stimulation corresponding to at least one stimulation unit may be provided to the user.

Here, the plurality of stimulation units is provided in a type embedded in the biofeedback device, and the plurality of stimulation units may include at least two of a vibration stimulation unit capable of providing a vibration stimulation, a sound stimulation unit capable of providing a sound stimulation, a light stimulation unit capable of providing a light stimulation, and an odor stimulation unit capable of providing an odor stimulation.

Further, the odor stimulation unit may include a plurality of fragrance storage units storing a plurality of fragrance materials, and an injection nozzle unit connected to the plurality of fragrance storage units and injecting any one of fragrance materials stored in the plurality of fragrance storage units by the control of the control unit.

Further, in step S13, the control unit may differently control a stimulation providing type of the stimulation provided from at least one stimulation unit by considering the heart rate identified in step S12 (in accordance with the heart rate).

Further, in step S13, when the heart rate satisfies a predetermined reference, the control unit may control the stimulation providing type of the stimulation provided by at least one stimulation unit to be changed from a first stimulation providing type to a second stimulation providing type.

Further, in step S13, when the heart rate satisfies the predetermined reference, the control unit may control an operation of the injection nozzle unit to inject a fragrance material stored in any one fragrance storage unit among a plurality of fragrance storage units. Further, when the heart rate does not satisfy the predetermined reference, the control unit may control the operation of the injection nozzle unit to inject the fragrance material stored in any one fragrance storage unit other than the any one fragrance storage unit among the plurality of fragrance storage units.

In the above description, steps S11 to S13 may be further divided into additional steps or combined into fewer steps, according to an embodiment of the present invention. In addition, some steps may be omitted as necessary, and the order between the steps may be changed.

Meanwhile, the control method of the biofeedback device using the electrocardiogram according to an embodiment of the present invention is implemented in a form of a program command which may be performed through various computer means and may be recorded in the computer readable medium. The computer readable medium may include a program command, a data file, a data structure, etc., singly or combinationally. The program command recorded in the medium may be specially designed and configured for the present invention or may be publicly known to and used by those skilled in the computer software field. An example of the computer readable recording medium includes magnetic media, such as a hard disk, a floppy disk, and a magnetic tape, optical media such as a CD-ROM and a DVD, magneto-optical media such as a floptical disk, and hardware devices such as a ROM, a RAM, and a flash memory, which are specially configured to store and execute the program command. An example of the program command includes a high-level language code executable by a computer by using an interpreter and the like, as well as a machine language code created by a compiler. The hardware device may be configured to be operated with one or more software modules in order to perform the operation of the present invention and vice versa.

Further, the control method of the biofeedback device using the electrocardiogram may be implemented even in the form of computer programs or applications to be executed by a computer, which are stored in the recording medium.

The aforementioned description of the present invention is used for exemplification, and it can be understood by those skilled in the art that the present invention can be easily modified in other detailed forms without changing the technical spirit or requisite features of the present invention. Therefore, it should be appreciated that the aforementioned embodiments are illustrative in all aspects and are not restricted. For example, respective constituent elements described as single types can be distributed and implemented, and similarly, constituent elements described to be distributed can also be implemented in a coupled form.

The scope of the present invention is represented by claims to be described below rather than the detailed description, and it is to be interpreted that the meaning and scope of the claims and all the changes or modified forms derived from the equivalents thereof come within the scope of the present invention.

## Claims

1. A biofeedback device using an electrocardiogram, comprising:
a signal acquisition unit acquiring an electrocardiogram signal of a user from an electrocardiograph;
an analysis unit analyzing the acquired electrocardiogram signal and providing a state diagnosis result of the user as an analysis result; and
a control unit controlling at least one stimulation unit among a plurality of stimulation units for state control of the user based on the state diagnosis result,
wherein a stimulation corresponding to the at least one stimulation unit is provided to the user by the control of the control unit.

2. The biofeedback device using an electrocardiogram of claim 1, wherein the plurality of stimulation units
is provided in a type embedded in the biofeedback device, and
includes at least two of a vibration stimulation unit capable of providing a vibration stimulation, a sound stimulation unit capable of providing a sound stimulation, a light stimulation unit capable of providing a light stimulation, and an odor stimulation unit capable of providing an odor stimulation.

3. The biofeedback device using an electrocardiogram of claim 1, wherein when there is a waveform which satisfies a predetermined feature condition within a waveform of the acquired electrocardiogram signal, the analysis unit identifies that a signal corresponding to the waveform satisfying the predetermined feature condition is an abnormal suspicious electrocardiogram signal.

4. The biofeedback device using an electrocardiogram of claim 3, wherein the analysis unit finally identifies whether the abnormal suspicious electrocardiogram signal is an abnormal electrocardiogram signal based on a comparison of a similarity between waveform data of a plurality of abnormal electrocardiogram signals prestored in a database and waveform data of the abnormal suspicious ECG signal to provide the state diagnosis result.

5. The biofeedback device using an electrocardiogram of claim 2, wherein the analysis unit identifies a heart rate of the user by analyzing the electrocardiogram signal, and
the control unit
differently controls a stimulation providing type of the stimulation provided from the at least one stimulation unit by considering the identified heart rate, and
controls the stimulation providing type of the stimulation provided by the at least one stimulation unit to be changed from a first stimulation providing type to a second stimulation providing type when the heart rate satisfies the predetermined reference.

6. The biofeedback device using an electrocardiogram of claim 5, wherein the odor stimulation unit includes
a plurality of fragrance storage units storing a plurality of types of fragrance materials, and
an injection nozzle unit connected to the plurality of fragrance storage units, and injecting any one of fragrance materials stored in the plurality of fragrance storage units by the control of the control unit.

7. The biofeedback device using an electrocardiogram of claim 6,
wherein the control unit
controls an operation of the injection nozzle unit to inject a fragrance material stored in any one fragrance storage unit among the plurality of fragrance storage units when the heart rate satisfies the predetermined reference, and
controls the operation of the injection nozzle unit to inject the fragrance material stored in any one fragrance storage unit other than the any one fragrance storage unit among the plurality of fragrance storage units when the heart rate does not satisfy the predetermined reference.

8. A control method of a biofeedback device using an electrocardiogram of claim 1, comprising:
acquiring, by a signal acquisition unit, an electrocardiogram signal of a user from an electrocardiograph;
analyzing, by an analysis unit, the acquired electrocardiogram signal and providing a state diagnosis result of the user as an analysis result; and
controlling, by a control unit, at least one stimulation unit among a plurality of stimulation units for the biofeedback for state control of the user based on the state diagnosis result,
wherein a stimulation corresponding to the at least one stimulation unit is provided to the user by the controlling.
